# EUROPEAN PATENT APPLICATION

(11) **EP 0 726 314 A1**
(43) Date of publication of application: **14.08.1996**
(21) Application number: 94929022.5
(22) Date of filing: 07.10.1994
(51) Int. Cl.: C12N 15/31, C12P 21/02, C12N 1/21, C07K 14/195, C07K 16/12, A61K 38/16, A61K 39/395

(54) **FIMBRILLIN PROTEIN OF $i(PORPHYROMONAS GINGIVALIS)**

(30) Priority: 08.10.1993 JP 253203/93
(71) Applicant: LION CORPORATION, Sumida-ku, Tokyo 130 (JP)
(72) Inventor: MORISHIMA, Seiji, Kanagawa 250 (JP)
(74) Representative: Adams, William Gordon
(86) International application number: JP9401687
(87) International publication number: WO9510612

(57) **Abstract**

A part or the whole of a nucleic acid encoding the fimbrillin protein of Porphyromonas gingivalis; an antigen comprising a part or the whole of the above protein; an antibody against the above antigen; and a medicine containing the above antibody. The above acid and antibody are useful for detecting P. gingivalis, and the antibody is useful for preventing or ameliorating periodontal diseases.

## Description

### TECHNICAL FIELD

The present invention relates to fimbrillin, which is the major fimbria structural protein of *Porphyromonas gingivalis* (hereunder referred to as *P*. *gingivalis*) and fragments thereof, to DNA encoding the fimbrillin, and to fragments thereof. Of such DNA fragments, those with high homology among the bacterial strains are useful for detecting the species, while those with low homology among the bacterial strains are useful as probes for distinguishing between the bacterial strains. Also, the proteins or their fragments which have high homology among the bacterial strains are useful as antigens for the production of antibodies which recognize the above-mentioned bacterial species, while peptides with low homology among the bacterial strains are useful as antigens for producing antibodies capable of distinguishing between the bacterial strains.

The present invention further relates to antibodies with immunoactivity against serum type-common peptide fragments of fimbrillin, the fimbria structural protein of *P*. *gingivalis*. These antibodies against common peptides may be used for prevention or improvement of periodontal disease by their common inhibition of fimbria function of different serum types.

### BACKGROUND ART

*P*. *gingivali*s, a gram-negative anaerobic bacterial species, has been the focus of attention in recent years as a periodontal pathogenic bacterium, due to its frequent detection in lesions of periodontal patients. The surface layer of the bacteria is known to contain fimbriae which have very high antigenicity. The fimbriae exhibit diverse biological activity against host cells, and are also highly implicated in the pathogenicity of *P*. *gingivalis*, as an adhesive factor.

The structure of the fimbrial protein is believed to comprise a subunit protein called fimbrillin with a molecular weight of about 41,000, which is polymerized into some shape.

The only base sequence coding for fimbrillin so far reported is that of *P*. *gingivalis* strain 381 [J. Bacteriol. 170, 1658 (1988)]. Recently, however, it has become evident that the immunological reactivity of the fimbriae differ among strains [Oral Microbiol. Immunol. 6, 332 (1991)], suggesting that this fimbrial protein is not consistent among *P*. *gingivalis* strains.

Thus, it has been a goal to elucidate the genetic sequences coding for fimbrillin of *P*. *gingivalis* with different immunological reactivities, and clarify the differences between bacterial strains.

However, inhibition of the pathogenicity of *Porphyromonas gingivalis* using antibodies against the fimbriae of the *Porphyromonas gingivalis* with different immunological reactivities requires antibodies against the fimbriae of each serum type, which is not feasible.

However, it is believed possible to inhibit fimbrial function through reaction of a single antibody with fimbriae of *Porphyromonas gingivalis* with different immunological reactivities, by using an antibody which is specific to a sequence common to their fimbrillin proteins.

### DISCLOSURE OF THE INVENTION

It is an object of the present invention, therefore, to provide DNA coding for the fimbrillin proteins of different strains of *P*. *gingivalis*, DNA fragments with high homology among the bacterial strains which are useful as probes for detecting the above-mentioned species, and DNA fragments with low homology among the bacterial strains (strain-specific) which are useful as probes for distinguishing between strains; as well as fimbrillin proteins from different strains having specific amino acid sequences, peptides having amino acid sequences with high homology among the bacterial strains which are useful for producing antibodies recognizing the species, and peptides having amino acid sequences with low homology among the bacterial strains (strain-specific) which are useful for producing antibodies capable of distinguishing between strains.

It is a further object of the present invention to provide antibodies capable of commonly reacting with these *Porphyromonas gingivalis* strains, by using antigens which are peptide fragments selected from amino acid sequence portions with high homology among the amino acid sequences of fimbrillin proteins of *Porphyromonas gingivalis* with different immunological reactivities, or the same peptide fragments bound to suitable proteins.

In order to accomplish the above-mentioned objects, the present invention provides DNA coding for the fimbrillin proteins of *Porphyromonas gingivalis* strains ATCC33277, BH18/10, HW24D-1, OMZ314, OMZ409, ATCC49417, 6/26 and HG564, which are included in the base sequences represented by Sequence Nos. 2 through 9.

The present invention further provides DNA having any base sequence forming a region comprising at least 10 contiguous bases, with at least 50% base sequence homology between at least 2 different base sequences of the base sequences listed as Sequence Nos. 1 through 9.

The present invention further provides DNA having any base sequence forming a region comprising at least 10 contiguous bases, with less than 50% base sequence homology between at least 2 different base sequences of the base sequences listed as Sequence Nos. 1 through 9.

The present invention further provides fimbrillin proteins of *Porphyromonas gingivalis* having the amino acid sequences listed as Sequence Nos. 11 through 18.

The present invention further provides a peptide having any amino acid sequence forming a region comprising at least 5 contiguous amino acids, with at least 50% amino acid sequence homology between at least 2 different amino acid sequences of the amino acid sequences listed as Sequence Nos. 10 through 18.

The present invention further provides a peptide having any amino acid sequence forming a region comprising at least 5 continuously linked amino acids, with less than 50% amino acid sequence homology between at least 2 different amino acid sequences of the amino acid sequences listed as Sequence Nos. 10 through 18.

The present invention further provides expression vectors comprising the different DNA described above.

The present invention further provides hosts containing the above-mentioned expression vectors.

The present invention further provides antibodies which react with fimbrillin of different strains of *Porphyromonas gingivalis*. Thus, according to the invention, antibodies are produced which react with *Porphyromonas gingivalis* of different serum types, by cloning fimbrillin-encoding genes from 9 *Porphyromonas gingivalis* strains, deducing the amino acid sequences for the fimbrillins from the base sequences of those genes, comparing the amino acid sequences to discover an amino acid sequence common to the plurality thereof, and preparing an antibody against the peptide having the common amino acid sequence.

The present invention, therefore, relates to antigenic peptides comprising at least 5 linked amino acids selected from the following amino acid sequences which are common to the amino acid sequences for fimbrillins of 9 strains of *Porphyromonas gingivalis*:

Sequence (1) Asn (or Lys) Gly Glu Gln Gln Glu Ala Ile
Lys Ser Ala (or Val) Glu Asn Ala Thr (or Ile) Lys Val Glu
Asp (or Asn) Ile Lys Cys Ser (or Gly) (Sequence No.: 21)
Sequence (2) Glu Asp (or Asn) Ile Lys Cys Ser (or Gly)
Ala Gly Gln Arg Thr Leu Val Val Met Ala Asn Thr Gly Ala
(or Glu, Gly) Met Glu (or Lys) Leu Val (or Ala) Gly Lys
Thr Leu Ala (Sequence No: 22)

Sequence (3) Val (or Ala) Gly Lys Thr Leu Ala Glu Val
Lys Ala Leu Thr Thr Glu Leu Thr Ala (or Glu) Glu (or Gly)
Asn Gln Glu Ala Ala Gly Leu Ile Met Thr Ala Glu Pro
(Sequence No: 23)
Sequence (4) Gln Gly Phe Tyr Val Leu Glu Asn (or Ser)
Thr (or Asp, Lys) Tyr Ala (or Ser, Asp) Gln (or Ala) Asn
(or Ser) (Sequence No.: 24)
Sequence (5) Gly (or Pro) Thr (or Lys) Thr Tyr Tyr Pro
Val Leu Val Asn Phe (or Tyr) Asn (or Glu, Asp) Ser (or
Gly) Asn Asn (or Gly) Tyr Thr (or Ile) Tyr (Sequence No.:
25)
Sequence (6) Ser Asn Asn Tyr Thr Tyr Asp Ser (or Asn)
Asn (or Gly) Tyr Thr Pro Lys Asn Lys Ile Glu Arg Asn His
Lys Tyr Asp Ile Lys Leu Thr Ile Thr Gly Pro Gly Thr Asn
(Sequence No.: 26)
Sequence (7) Ile Thr Gly Pro Gly Thr Asn Asn (or Thr)
Pro Glu Asn Pro Ile (or Gln) Thr Glu Ser Ala His (or Asn)
Leu Asn Val Gln Cys Thr Val Ala Glu Trp Val Leu Val Gly
Gln Asn Ala Thr Trp (Sequence No.: 27)
Sequence (8) Thr Gly Ser Leu Thr Thr (or Asn) Phe Asn
Gly Ala Tyr Ser (or Thr) Pro Ala Asn Tyr Thr (or Ala)
(Sequence No.: 28)

The present invention further relates to antigens which are the above-mentioned peptides or carrier/protein complexes.

The present invention further relates to antibodies against the above-mentioned antigenic peptides or antigen complexes, and this encompasses both polyclonal antibodies and monoclonal antibodies.

The present invention further relates to an agent for the prevention or improvement of periodontal diseases which contains any of the above-mentioned antibodies.

### BRIEF DESCRIPTION OF THE DRAWINGS

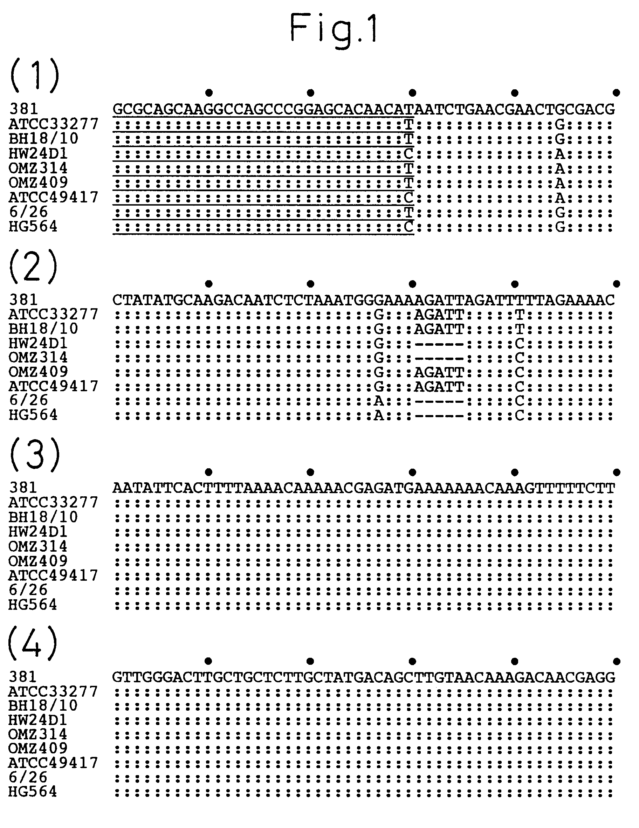

Fig. 1 is a sequence list aligning the mutually corresponding parts of the base sequences of the genes coding for fimbrillins of the 9 strains of *P. gingivalis*. The underlined portions indicate the PCR primer sequence. The sites indicated by the colon ":" are the same in all 9 strains, and "-" indicates gaps produced to obtain the best matching position.

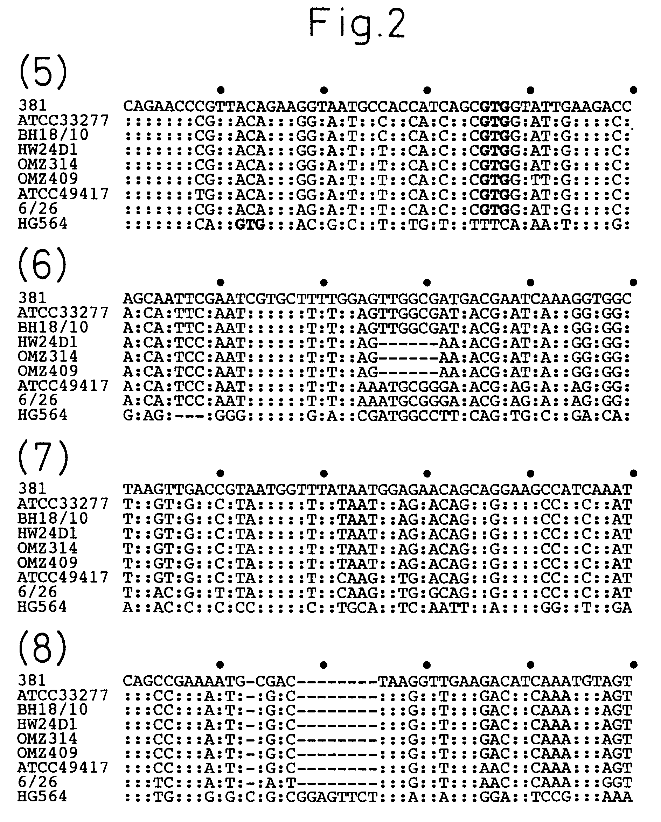

Fig. 2 is a sequence list aligning the mutually corresponding parts of the base sequences of the genes coding for fimbrillins of the 9 strains of *P*. *gingivalis*. The sites indicated by the colon ":" are the same in all 9 strains, and "-" indicates gaps produced to obtain the best matching position.

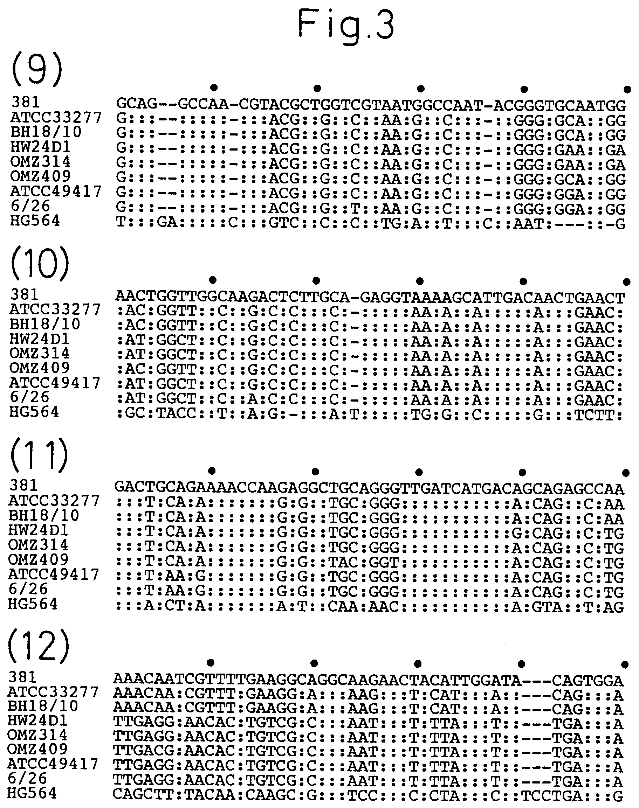

Fig. 3 is a sequence list aligning the mutually corresponding parts of the base sequences of the genes coding for fimbrillins of the 9 strains of *P*. *gingivalis*. The sites indicated by the colon ":" are the same in all 9 strains, and "-" indicates gaps produced to obtain the best matching position.

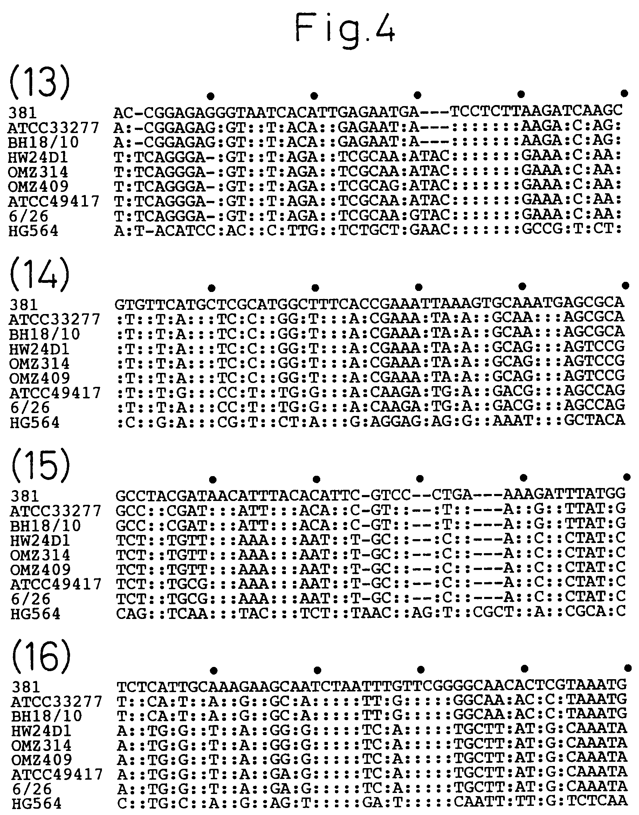

Fig. 4 is a sequence list aligning the mutually corresponding parts of the base sequences of the genes coding for fimbrillins of the 9 strains of *P*. *gingivalis*. The sites indicated by the colon ":" are the same in all 9 strains, and "-" indicates gaps produced to obtain the best matching position.

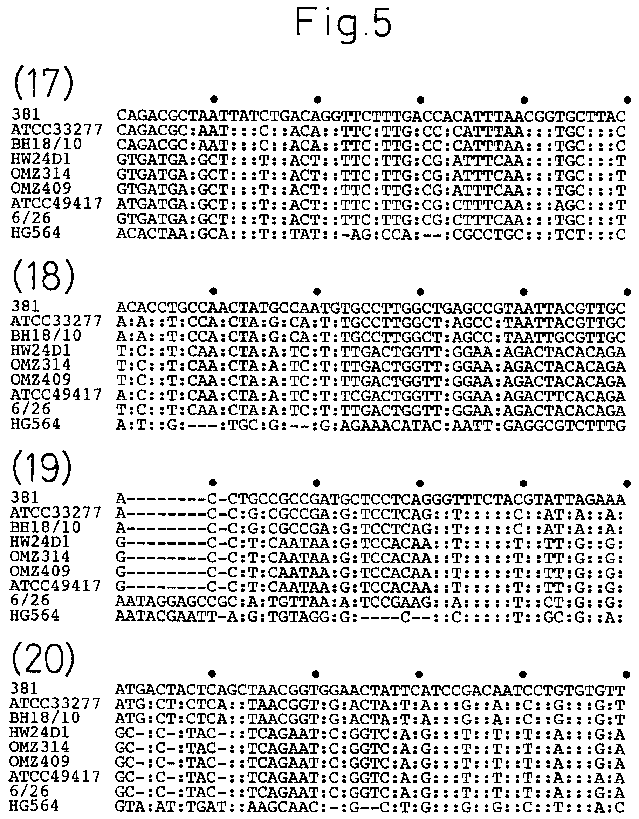

Fig. 5 is a sequence list aligning the mutually corresponding parts of the base sequences of the genes coding for fimbrillins of the 9 strains of *P*. *gingivalis*. The sites indicated by the colon ":" are the same in all 9 strains, and "-" indicates gaps produced to obtain the best matching position.

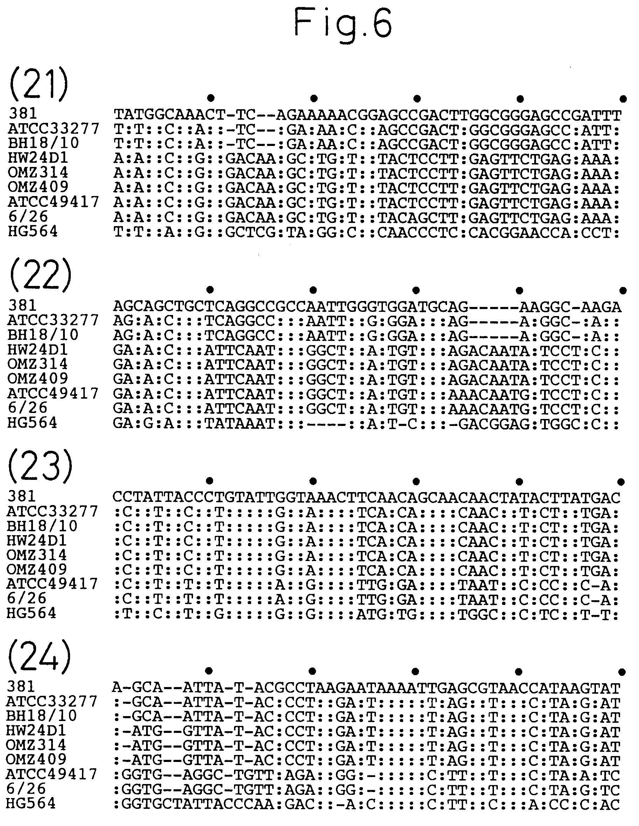

Fig. 6 is a sequence list aligning the mutually corresponding parts of the base sequences of the genes coding for fimbrillins of the 9 strains of *P*. *gingivalis*. The sites indicated by the colon ":" are the same in all 9 strains, and "-" indicates gaps produced to obtain the best matching position.

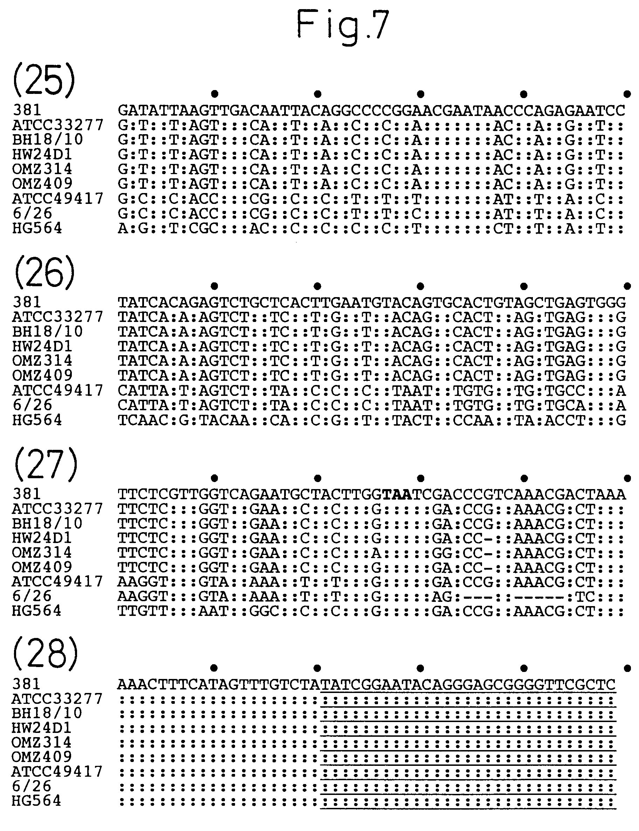

Fig. 7 is a sequence list aligning the mutually corresponding parts of the base sequences of the genes coding for fimbrillins of the 9 strains of *P*. *gingivalis*. The underlined portions indicate the PCR primer sequence. The sites indicated by the colon ":" are the same in all 9 strains, and "-" indicates gaps produced to obtain the best matching position.

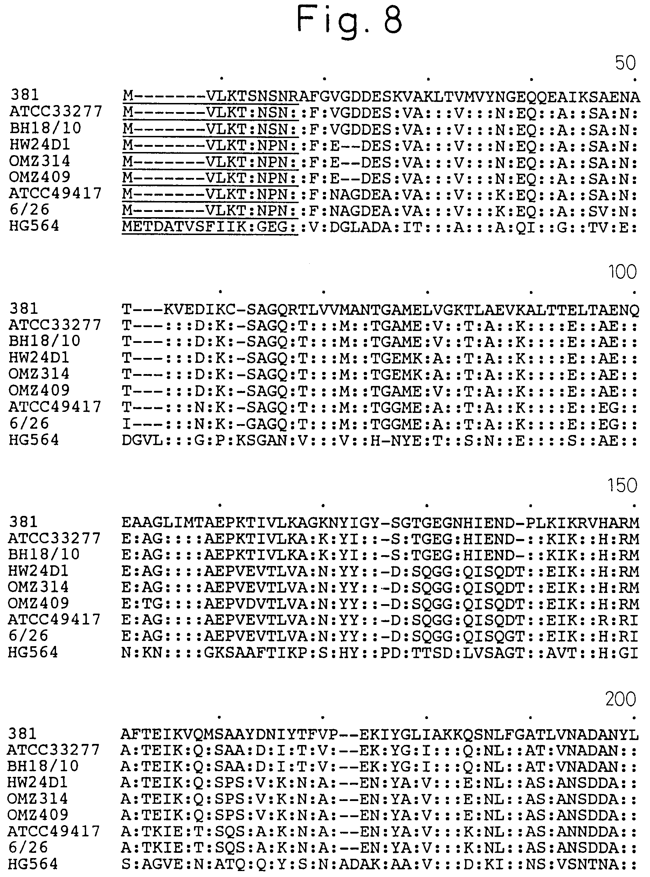

Fig. 8 is a sequence list aligning the mutually corresponding parts of the amino acids sequences of fimbrillins of the 9 strains of *P*. *gingivalis*. The underlined portions indicate the position of the signal peptide. The sites indicated by the colon ":" are the same in all 9 strains, and "-" indicates gaps produced to obtain the best matching position.

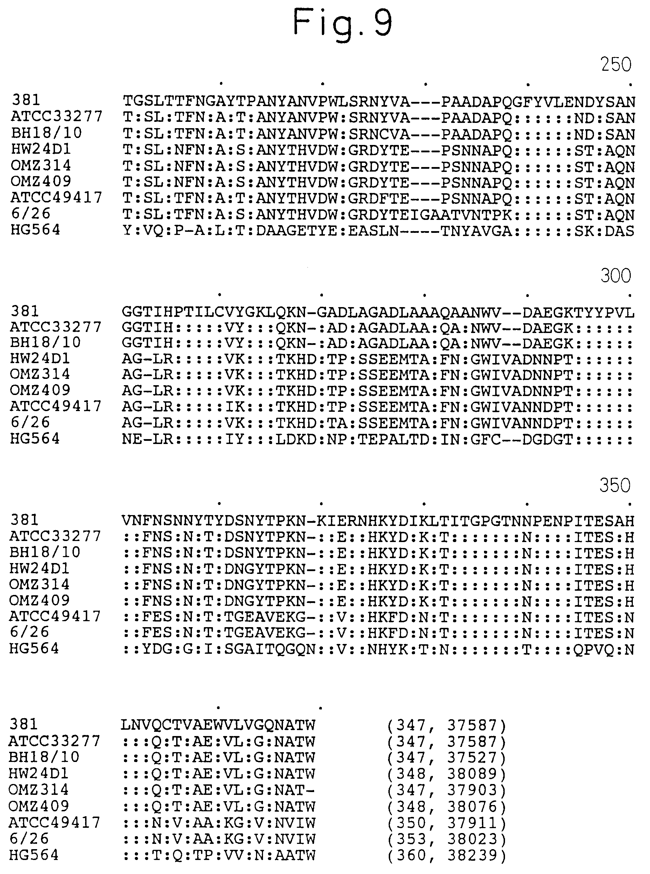

Fig. 9 is a sequence list aligning the mutually corresponding parts of the amino acids sequences of fimbrillins of the 9 strains of *P*. *gingivalis*. The sites indicated by the colon ":" are the same in all 9 strains, and "-" indicates gaps produced to obtain the best matching position.

### DETAILED DESCRIPTION OF THE INVENTION

The present invention first provides novel DNA encoding fimbrillin proteins of different strains of *P*. *gingivalis*, and novel fimbrillin proteins encoded by the DNA.

According to the invention, *P*. *gingivalis* strains 381, ATCC33277, BH18/10, HW24D-1, OMZ314, OMZ409, ATCC49417, 6/26 and HG564 are used, chromosomal DNA from these 9 strains is purified from the bacteria and used as templates in the PCR (polymerase chain reaction) to amplify DNA containing the genes (fimA) coding for fimbrillin. The amplified fimA genes from these 9 strains are cloned, and their base sequences and amino acid sequences are determined.

As a more detailed explanation of the invention, the chromosomal DNA from *P*. *gingivalis* may be obtained by extraction and purification from cultured cells, etc. The chromosomal DNA is used as the template in the PCR to amplify the DNA containing the fimA gene. The PCR primer used is an oligonucleotide of appropriate length synthesized and purified with a DNA synthesizer based on the DNA sequence of the already publicly known *P*. *gingivalis* 381 fimA gene. Here, a restriction endonuclease-recognizing sequence not present in the fimA gene is preferably artificially added to facilitate cloning of the amplified PCR product in a vector plasmid.

After digesting the DNA amplified in this manner with the restriction endonuclease added to the primer, DNA ligase may be used to link it downstream of the promoter of a suitable expression vector which has been digested with the same enzyme, and this recombinant DNA may be introduced into *E*. *coli* or the like to obtain the desired clone. Here, a vector system which allows modification of expression of the protein encoded by the inserted gene is preferably used, such as one including the repressor for the promoter of an expression vector such as lacI^{q}, in either or both the plasmid vector and the DNA of the *E*. *coli* host.

In order to obtain the desired clones from the transformant obtained in this manner, screening may be performed by a method such as colony hybridization, using the fimA gene or a fragment thereof labelled with a suitable compound as the probe.

Also, it may be determined whether or not the obtained clones are the desired ones by extracting and separating the plasmid DNA from the transformed strains, digesting it with a suitable restriction enzyme and then identifying it by agarose gel electrophoresis.

It is even more reliable to confirm expression of the recombinant fimbrillin with a method such as Western blotting using anti-*P*. *gingivalis* fimbria antibody, after the *E*. *coli* clones have been induced to express the recombinant proteins.

The base sequences of the *P*. *gingivalis* fimA genes cloned in this manner may be determined in the same manner as described above, by digestion with a suitable restriction enzyme followed by incorporation of a suitable vector, preparation of deletion mutant strains based on the method of Steven Henikoff, extraction and purification of plasmid DNA from the series of deletion mutants, or infection with a suitable helper phage and purification of the single-stranded DNA, and then determination of the base sequence based on the Sanger method.

The results of determining the base sequences are shown as Sequence Nos. 1 to 9. Sequence No. 1 shows the base sequence of a DNA fragment containing the base sequence coding for fimbrillin of strain 381, Sequence No. 2 for that of strain ATCC33277, Sequence No. 3 for that of strain BH18/10, Sequence No. 4 for that of strain HW24D-1, Sequence No. 5 for that of strain OMZ314, Sequence No. 6 for that of strain OMZ409, Sequence No. 7 for that of strain ATCC49417, Sequence No. 8 for that of strain 6/26 and Sequence No. 9 for that of strain HG564.

In addition, the amino acid sequences encoded by the reading frames of these base sequences are shown in Sequence Nos. 10 to 18. That is, Sequence No. 10 shows the amino acid sequence for fimbrillin of strain 381, Sequence No. 11 for that of strain ATCC33277, sequence No. 12 for that of strain BH18/10, Sequence No. 13 for that of strain HW24D-1, Sequence No. 14 for that of strain OMZ314, Sequence No. 15 for that of strain OMZ409, Sequence No. 16 for that of strain ATCC49417, Sequence No. 17 for that of strain 6/26 and Sequence No. 18 for that of strain HG564.

When the present invention is applied for the identification of *P*. *gingivalis* or a strain thereof, one of the base sequences listed as Sequence Nos. 1 to 9 or a fragment thereof, of DNA having a substantially identical base sequence, is preferred. Here, "substantially identical" means a sequence which is homologous enough with any of the above-mentioned base sequences so as to be able to distinguish the type or strain of interest. However, the DNA used for production of the fimbrillin protein or partial peptide thereof by recombination may have any desired codons coding for the amino acid sequences listed as Sequence Nos. 10 to 18 or portions thereof.

The above-mentioned DNA or fragment thereof is obtained by cloning from the different strains mentioned above, with cleavage or modification, as necessary. Once the base sequence has been determined, however, the desired DNA may be chemically synthesized according to a well-known method. The DNA of the invention may thus be obtained even if the above-mentioned strains are not available.

According to one embodiment of the present invention, a DNA fragment forming a region with high homology among the aforementioned plurality of base sequences may be used as a probe for detection of *P*. *gingivalis*. Such a DNA fragment may be a DNA fragment in a region comprising at least 10 contiguous bases having at least 50% homology between any 2 different base sequences of the above-mentioned 9 base sequences. The homology is preferably at least 70%, more preferably at least 80% and most preferably at least 90%. The homologous region preferably consists of 20 bases or more.

Such a homologous region may be easily determined by observation of the aligned base sequences, or using an available computer program. According to the invention, "homology" is defined as the ratio of the number of identical bases or amino acids between two different sequences with respect to their total number of bases or amino acids, upon comparison of the base sequences or amino acid sequences of given corresponding regions of the two base sequences or amino acid sequences.

According to another embodiment of the invention, there are provided DNA fragments consisting of regions with low homology among a plurality of base sequences, i.e. with high strain specificity. Such DNA fragments are useful as probes for distinguishing strains belonging to *P*. *gingivalis*. Such a DNA fragment may be a DNA fragment consisting of a base sequence of at least 10 contiguous bases having less than 50% homology between any 2 different base sequences of the base sequences listed as Sequence Nos. 1 to 9. The homology is preferably 40% or less, more preferably 30% or less and most preferably 20% or less. The length of the continuous non-homologous base sequence is preferably 20 bases or more.

The DNA used as the probe is preferably labelled with commonly used labelling such as radioactive labelling, fluorescent labelling or enzyme labelling. Labelling types, labelling methods and labelling detection methods are a well-known technology in this field.

The present invention further provides peptides having amino acid sequences forming regions with high homology among a plurality of the amino acid sequences listed as Sequence Nos. 10 to 18. Such peptides are useful as antigens for production of antibodies, for example, polyclonal or monoclonal antibodies, which recognize fimbrillin of *P*. *gingivalis.*

Such a peptide may be a peptide with an amino acid sequence of at least 5 contiguous amino acids having at least 50% homology between any 2 different amino acid sequences of the amino acid sequences listed as Sequence Nos. 10 to 18. The sequence preferably has at least 70%, and more preferably at least 90% homology. The length of the continuous amino acid sequence is preferably 10 amino acids or more.

The present invention further provides peptides consisting of amino acid sequences in regions with low homology among a plurality of the amino acid sequences listed as Sequence Nos. 10 to 18, i.e. with high strain specificity. Such peptides are useful as antigens for production of antibodies, i.e. polyclonal or monoclonal antibodies, capable of distinguishing fimbrillin of different *P*. *gingivalis* strains. Such a peptide may be a peptide in a region consisting of at least 5 contiguous amino acids with less than 50% homology between any 2 different amino acid sequences of the amino acid sequences listed as Sequence Nos. 10 to 18. The homology is preferably 40% or less, and more preferably 30% or less.

These peptides are useful as antigens for producing polyclonal or monoclonal antibodies by common methods, and may be used by themselves or bound with other proteins. For example, on of the above-mentioned proteins or peptides may be used as an antigen, either alone or coupled with another protein, for immunization of a suitable mammal or bird, to obtain a fimbrillin-specific antibody. The antibody may then be labelled with a suitable labelling compound in the same manner as the oligonucleotide primer described earlier, and used to detect a specific *P*. *gingivalis.*

Such polypeptides or their coupled forms with proteins such as BSA may themselves be used as vaccine antigens, or the specific antibodies as immunoactive antibodies, for the prevention or curing of periodontal diseases caused by *P*. *gingivalis.*

A protein or peptide having an amino acid sequence as described above may be prepared by a commonly employed gene recombinant method, or by a common method of chemical synthesis. Thus, the present invention also provides expression vectors comprising the above-mentioned DNA, hosts transformed by those expression vectors, and a method of producing desired protein kinases using those hosts. Therefore, both prokaryotic and eukaryotic hosts may be used.

Prokaryotic hosts which may be used include bacteria, for example *Escherichia coli* and *Bacillus* microorganisms such as *Bacillus subtilis*. The eukaryotic host may be a lower eukaryote, for example yeast including *Saccharomyces* yeast, such as *Saccharomyces cerevisiae*.

Animal cells may be used as higher eukaryotic hosts, for example CHO cells, Hela cells and COS cells. Insect cells such as silk worm cells or Mamestra cells may also be used. Insect imagos may be used, as well.

The expression vector used may be a plasmid, phagmid, phage or virus, depending on the host. For example, plasmids or phagmids are used for bacterial hosts, plasmids are used for yeast hosts, and viruses such as vaccinia virus or baculovirus are used for animal or insect cells.

The expression vector includes, in addition to the structural gene coding for the above-mentioned protein kinase, expression regulating regions which are operably linked to the structural gene, for example a promoter, enhancer, terminator, etc. Examples of promoters used for bacteria are tryptophan operon, Tac promoter and Trc promoter; examples of promoters used for yeast are TDH₃ promoter, ADHI promoter, GALI-7 promoter and PGKL promoter; examples of promoters used for animal cells are SV40 promoter, Ad2 promoter and vaccinia 75K promoter; and an example of a promoter used for insect cells is polyhedrin promoter.

The culturing of the transformed host and expression of the desired gene may be carried out by common methods depending on which host, promoter, etc. is used. Also, the isolation and purification of the desired protein from the cultured product may be accomplished by an appropriate combination of common methods for isolation and purification of proteins, such as filtration, centrifugation, salting out, column chromatography, electrophoresis, affinity chromatography, and the like.

The detection of *P*. *gingivalis* using a DNA or peptide according to the invention may be accomplished, for example, in the following manner.

### Detection of desired strain using DNA probe

Detection of a desired strain in a test sample may be accomplished by immobilizing a DNA sample, which has undergone suitable treatment, on a nylon membrane or other support and hybridizing it with a DNA probe already labelled with a labelling compound which is suitable to the purpose, and after removing the non-specifically binding labelled DNA probe, detecting the DNA probe.

The DNA probe used here may be one which has been cloned from *P*. *gingivalis* chromosomal DNA and digested with a suitable restriction endonuclease, or one which has been chemically synthesized with a DNA synthesizer.

The method of labelling the DNA probe may be, for example, the method according Molecular Cloning: A Laboratory Manual, Cold Spring Harbor Laboratory Press (1989) using a radioactive compound such as ³²P, or according to Nucl. Acids Res. 14, 6115 (1986) using alkaline phosphatase.

The general method for detecting the probe may be by autoradiography, coloration reaction, etc., and quantitative detection of the desired strain is also possible by detecting the probe bound to the DNA of the desired strain.

### Detection of desired strain using specific antibody

Detection of a desired strain using an antibody specific to the fimbrillin or constituent peptide thereof is possible by employing the general detection method for antigens based on antigen-antibody reaction.

Examples of methods which may be used include the ELISA method, latex agglutination method, immunoelectrophoresis method, Ouchterlony method, etc. The test samples used in these methods may be reacted after pretreatment by heating, etc. either directly or in a suitable solvent, and the amount of the resulting antigen-antibody complex may be directly or indirectly quantitated for quantitation of the desired strain.

In order to obtain an antibody which reacts with fimbrillin proteins of different strains of *Porphyromonas gingivalis*, it is necessary to obtain an antibody against a peptide with an amino acid sequence common to those fimbrillin proteins. Upon comparing the amino acid sequences deduced from DNA coding for fimbrillin proteins of 9 different cloned strains according to the invention, the following 8 representative common sequences were found to exist.

Sequence (1) Asn (or Lys) Gly Glu Gln Gln Glu Ala Ile
Lys Ser Ala (or Val) Glu Asn Ala Thr (or Ile) Lys Val Glu
Asp (or Asn) Ile Lys Cys Ser (or Gly) (Sequence No.: 21)
Sequence (2) Glu Asp (or Asn) Ile Lys Cys Ser (or Gly)
Ala Gly Gln Arg Thr Leu Val Val Met Ala Asn Thr Gly Ala
(or Glu, Gly) Met Glu (or Lys) Leu Val (or Ala) Gly Lys
Thr Leu Ala (Sequence No: 22)
Sequence (3) Val (or Ala) Gly Lys Thr Leu Ala Glu Val
Lys Ala Leu Thr Thr Glu Leu Thr Ala (or Glu) Glu (or Gly)
Asn Gln Glu Ala Ala Gly Leu Ile Met Thr Ala Glu Pro
(Sequence No: 23)
Sequence (4) Gln Gly Phe Tyr Val Leu Glu Asn (or Ser)
Thr (or Asp, Lys) Tyr Ala (or Ser, Asp) Gln (or Ala) Asn
(or Ser) (Sequence No.: 24)
Sequence (5) Gly (or Pro) Thr (or Lys) Thr Tyr Tyr Pro
Val Leu Val Asn Phe (or Tyr) Asn (or Glu, Asp) Ser (or
Gly) Asn Asn (or Gly) Tyr Thr (or Ile) Tyr (Sequence No.:
25)
Sequence (6) Ser Asn Asn Tyr Thr Tyr Asp Ser (or Asn)
Asn (or Gly) Tyr Thr Pro Lys Asn Lys Ile Glu Arg Asn His
Lys Tyr Asp Ile Lys Leu Thr Ile Thr Gly Pro Gly Thr Asn
(Sequence No.: 26)
Sequence (7) Ile Thr Gly Pro Gly Thr Asn Asn (or Thr)
Pro Glu Asn Pro Ile (or Gln) Thr Glu Ser Ala His (or Asn)
Leu Asn Val Gln Cys Thr Val Ala Glu Trp Val Leu Val Gly
Gln Asn Ala Thr Trp (Sequence No.: 27)
Sequence (8) Thr Gly Ser Leu Thr Thr (or Asn) Phe Asn
Gly Ala Tyr Ser (or Thr) Pro Ala Asn Tyr Thr (or Ala)
(Sequence No.: 28)

The positions of the amino acid sequences of the above-mentioned Sequence Nos. 21 to 28 in the amino acid sequences listed as Sequence Nos. 10 to 18 are as shown in the following table.

**Table 1**

| Sequence No. | 21 | 22 | 23 | 24 | 25 | 26 | 27 | 28 |
|---|---|---|---|---|---|---|---|---|
| Sequence No. | | | | | | | | |
| 10 | 20-42 | 37-65 | 60-90 | 210-222 | 262-279 | 274-307 | 301-337 | 177-190 |
| 11 | 20-42 | 37-65 | 60-90 | 210-222 | 262-279 | 275-307 | 301-337 | 177-190 |
| 12 | 20-42 | 37-65 | 60-90 | 210-222 | 262-279 | 274-307 | 301-337 | 177-190 |
| 13 | 18-40 | 35-63 | 58-88 | 209-221 | 263-280 | 275-308 | 302-338 | 176-189 |
| 14 | 18-40 | 35-63 | 58-88 | 209-221 | 263-280 | 275-308 | 302-337 | 176-189 |
| 15 | 18-40 | 35-63 | 58-88 | 209-221 | 263-280 | 276-308 | 302-338 | 176-189 |
| 16 | 20-42 | 37-65 | 60-90 | 211-223 | 265-282 | 278-310 | 304-(324) | 178-191 |
| 17 | 20-42 | 37-65 | 60-90 | (214)-226 | 268-285 | 280-313 | 306-(327) | 178-191 |
| 18 | - | - | - | (216)-227 | 267-284 | - | 307-319 | |

The antigen peptides of the present invention are peptides comprising at least 5 contiguous amino acids of the above-mentioned amino acid sequences (Sequence Nos. 21-28). The other common peptides shown in Figs. 8 and 9 may also be used in the same manner. In cases where the peptides lack antigenicity due to short length, etc., they may be used as antigens by attachment to a carrier protein.

These peptides may be synthesized by common methods such as the solid phase method or liquid phase method. Alternatively, the peptides may be produced by expression of their encoding genes. The genes may be chemically synthesized or the DNA coding for the amino acid sequences listed as Sequence Nos. 10 to 18 or DNA having, for example, the base sequences listed as Sequences Nos. 1 to 9 may be used as templates to amplify a desired portion thereof by the PCR method. For amplification by PCR, the cleavage site of a restriction endonuclease is preferably bound artificially to the PCR primer.

These DNA themselves may be expressed to obtain the peptides, or they may be linked to DNA coding for other proteins and expressed to obtain fused proteins with other proteins. Such expression may be carried out by common methods.

The desired peptide or fused protein which is obtained in this manner may be used as an immunogen either alone or in the form of a complex with a suitable carrier protein. The type of carrier protein used here may be bovine serum albumin, egg albumin, myoglobin, tetanus toxoid, KLH (keyhole limpet hemocyanin), etc.

Also, the binding of the carrier protein to the peptide of interest may be accomplished using publicly known means. The reagent used for the binding may be, for example, glutaraldehyde, a bis-imido ester, bis-diazotized benzidine, soluble carbodiimide, m-maleimidobenzoyl-N-hydroxysuccinimide, or the like. The binding ratio (molar ratio) of the carrier protein to the peptide is preferably between 1:1 and 1:40, and especially between 1:5 and 1:20.

To obtain polyclonal antibodies, the immunogen obtained in this manner may be used to immunize a mammalian animal (sheep, goat, cow, horse, pig, rabbit, rat, mouse, guinea pig, etc.) or bird (chicken, dove, quail, duck, goose, etc.), and the antibodies obtained from the animal's serum, milk, ova, egg yolk, etc. Mammalian animals may be immunized by a normal method such as subcutaneous, intramuscular or intraabdominal administration of the obtained immunogen, or by nosedrops or eyedrops. If necessary it may be used for immunization in admixture with an adjuvant such as Freund's complete adjuvant. Here, the amount of antigen used per immunization is preferably 0.1-3 mg/kg body weight, and particularly 0.25-2 mg/kg body weight, but an appropriate amount may be selected which gives the desired antibody titer while not adversely affecting the animal.

The immunization may be carried out 3-5 times every 2 to 4 weeks, taking blood from the immunized animal by a common method at 10 to 14 days after the final immunization, to obtain antiserum. The obtained antiserum may be used directly or after treatment by an appropriate process such as salting out, dialysis, ion exchange chromatography, gel filtration, affinity chromatography, etc. to purify the desired immunoglobulin.

Also, though there are no particular restrictions on the bird used for immunization, egg-laying species such as white leghorn hens are preferred from the standpoint of antibody production. The method of administrating the immunogen may be the same as described above for mammalian animals, and an appropriate antigen dosage may be selected which gives the desired antibody titer while not adversely affecting the animal. If necessary, it may also be used for immunization in admixture with an adjuvant such as Freund's complete adjuvant.

Antibodies may usually be prepared from a bird immunized in this manner by extraction and separation of immunoglobulin contained in the egg yolk. The method used for the extraction and separation may be a commonly used method of extracting immunoglobulin, such as precipitation using polysaccharides or polyethylene glycol, or extraction using an organic solvent such as ethanol or chloroform, and purification may be accomplished using the method of purification from antiserum described above.

The antibodies may also be obtained and used as monoclonal antibodies. To obtain monoclonal antibodies, the aforementioned immunogen is preferably used to immunize an animal such as a mouse, rat, guinea pig, etc. by a similar method, extracting spleen cells at 2 to 5 days after the final immunization, and preparing monoclonal antibody-producing hybridomas according to a common method. The antibody-producing hybridomas obtained in this manner may be cultured in a suitable medium and monoclonal antibodies may be purified for use from the resulting culture supernatant using the same means as in the aforementioned method of purifying polyclonal antibodies.

The antiserum or antibody titer may be measured using the ELISA method or radioimmunoassay method which are usually employed.

The antiserum, polyclonal antibodies or monoclonal antibodies obtained in this manner react specifically with fimbriae on the surface layer of *Porphyromonas gingivalis*, and thus are immunoactive against *Porphyromonas gingivalis*. That is, the antibodies themselves, or the antibodies labelled with a suitable labelling compound, may be used to specifically detect *P*. *gingivalis*. The antibodies also have an effect of inhibiting adhesion of *Porphyromonas gingivalis* to intraoral tissue, and thus applying the antibodies intraorally will inhibit intraoral colonization by *Porphyromonas gingivalis* and help prevent periodontal diseases.

Thus, the antibodies according to the invention may be suitably used as active ingredients of prophylactic agents for periodontal disease, and may be combined with various formulations depending on the mode of administration to the oral cavity, for example, dental cream, liquid dentifrice, mouthwash, etc. Other additional publicly known active ingredients may also be combined with the prophylactic agent for periodontal disease according to the invention in addition to the above-mentioned antibodies, depending on the type of formulation.

### EXAMPLES

The present invention will now be explained in more detail by way of the following examples.

### Example 1: Cloning of DNA coding for fimbrillin protein

### Preparation of P. gingivalis chromosomal DNA

*P*. *gingivalis* strains 381, ATCC33277, BH18/10, HW24D1, OMZ314, OMZ409, ATCC49417, 6/26 and HG564 were anaerobically cultured for 2 days at 37°C in a GAM liquid medium containing hemin and menadione, and the cells collected from centrifugation were washed with a TE buffer (10 mM Tris·HCl, 1 mM EDTA; pH 8.0) and then dispersed in 20 ml of the same buffer.

To this was added 0.4 ml of diethyl pyrocarbonate and heating was conducted at 50°C for 1 hour, after which the cells were again collected by centrifugation and dispersed in 5 ml of a TEN buffer (10 mM Tris·HCl, 1 mM EDTA, 100 mM NaCl; pH 8.0), and then the cells were lysed by addition of lysozyme, SDS and N-lauroylsarcosine sodium to final concentrations of 1 mg, 10 mM and 2%, respectively. Next, proteinase K was added to 50 µg/ml and the mixture was heated at 55°C for one hour. The supernatant obtained from centrifugation was subjected to cesium chloride density gradient centrifugation to separate the chromosomal DNA which was then purified by dialysis against TE buffer.

### Amplification of fimA gene by PCR

The obtained *P*. *gingivalis* chromosomal DNA was used as a template for amplification of the fimA gene by PCR. That is, using the base sequence of the fimA gene of *P*. *gingivalis* strain 381 as the basis for the design, primers with the BamHI recognition site (underlined) added [5'-AATTGGATCCGCGCAGCAAGGCCAGCCCGG-3' (Sequence No.: 19) and 5'- AGAGGGATCCGAGCGAACCCCGCTCCCTGT-3' (Sequence No.: 20)] were used for 20-30 cycles of PCR with Taq DNA polymerase, to amplify DNA containing the fimA gene.

### Cloning of fimA gene

After the DNA amplified by PCR and the expression vector pTrc99 were digested with BamHI, T4 DNA ligase was used for linking. The resulting recombinant DNA was introduced into *E*. *coli* JM109 which was then cultured overnight on an LB (Luria-Bertani) agar medium containing 100 ug/ml ampicillin to obtain transformant strains.

### Screening of positive clones by colony hybridization

After transferring the transformant colonies onto a nylon membrane, the cells were lysed with SDS in the presence of an alkali, and then the ³²P-labelled fimA gene was used as a probe for colony hybridization to screen for the desired positive clones.

### Example 2: Expression of recombinant protein by Western blotting

In order to more absolutely confirm that the obtained clones were the desired ones, expression of the recombinant fimbrillin protein was confirmed by Western immunoblot. That is, the *E*. *coli* clones were shake cultured in LB liquid medium containing 100 µg/ml ampicillin until the OD₅₅₀ reached about 0.3-0.5, and then IPTG was added to 0.2-1 mM and culturing was continued for another hour or more. After the culturing, the cells were collected by centrifugation and subjected to SDS-PAGE, and Western blotting using anti-*P*. *gingivalis* fimbrillin serum confirmed expression of the recombinant protein at the position of approximate molecular weight 43,000-48,000.

### Example 3: Determination of fimA gene base sequence

A DNA fragment containing fimA was digested with BamHI from the desired plasmid DNA, purified, and linked to vector pUC119 using T4 DNA ligase. Two types were prepared, with insertion of the fimA gene in both the forward and reverse directions with respect to the lac promoter of pUC119, and after digestion with KpnI and SmaI, the methods of Steven Henikoff and Yanisch Perron, et al. were basically followed to prepare deletion mutant strains with sizes differing by 100-200 bp each, which were transformed in *E*. *coli* MV1184 as a series of clones.

The series of defective plasmids were extracted, purified and denatured from the *E*. *coli* clones, and after infecting the clones with M13 helper phage and extraction and purification of the single-stranded DNA, the base sequences of the fimA genes were determined by the Sanger method.

The sequences of fimA genes of 9 *P*. *gingivalis* strains were thus elucidated (Sequence Nos. 1-9). From these results, it was found that the DNA base sequences shown in the sequence list had GTG as the initiation codon at bases 216-218 of *P*. *gingivalis* strains 381, ATCC33277, BH18/10, OMZ409 and ATCC49417, at bases 211-213 of strains HW24D-1, OMZ314 and 6/26 and at bases 187-189 of strain HG564, and coded for the amino acids of Sequence Nos. 10-18 containing the signal sequence.

### Example 4: Preparation of antigen

### Preparation of desired peptides

Among the amino acid sequences of the above-mentioned 9 strains selected as amino acid sequence portions common to the fimbrillin proteins of the different *Porphyromonas gingivalis* strains, the following 4 different desired peptides (1) to (4):
(1) The amino acid sequence corresponding to amino acids 14-31 of Sequence No. 23 (amino acids 73-89 of Sequence No. 10) (Sequence No. 29): Glu Leu Thr Ala Glu Asn Gln Glu Ala Ala Gly Leu Ile Met Thr Ala Glu Pro
(2) The amino acid sequence corresponding to amino acids 2-15 of Sequence No. 28 (amino acids 177-190 of Sequence No. 10) (Sequence No. 30): Gly Ser Leu Thr Thr Phe Asn Gly Ala Tyr Ser Pro Ala Asn
(3) The amino acid sequence corresponding to amino acids 3-17 of Sequence No. 25 (amino acids 264-278 of Sequence No. 10) (Sequence No. 31): Thr Tyr Tyr Pro Val Leu Val Asn Phe Asn Ser Asn Asn Tyr Thr
(4) The amino acid sequence corresponding to amino acids 4-20 of Sequence No. 27 (amino acids 304-320 of Sequence No. 10) (Sequence No. 32): Pro Gly Thr Asn Asn Pro Glu Asn Pro Ile Thr Glu Ser Ala His Leu Asn were chemically synthesized according to a common method of solid phase synthesis using an automatic peptide synthesizer (product of Applied Biochemicals Co.), and then separated off by high performance liquid chromatography and desalted and purified by gel filtration.

By another method, DNA coding for the amino acid sequences of the desired peptides were amplified from chromosomal DNA by the PCR, and were introduced into suitable expression vectors and expressed. That is, PCR primers were designed from the desired DNA sequences. The BamHI site was artificially added to the forward primer and the EcoRI site to the reverse primer, with the synthesis performed using a DNA synthesizer.

These PCR primers were used for 30 cycles of PCR with the *Porphyromonas gingivalis* chromosomal DNA as the template, to obtain DNA fragments amplified by common methods. After digestion with BamHI and EcoRI, they were inserted at the same sites of vector pGEX-3X (product of Pharmacia Co.). The resultant chimeric plasmids were used to transform *E*. *coli* JM109 by a common method, and the desired *E*. *coli* clones were obtained.

The *E*. *coli* clones were shake cultured in LB medium until the OD₅₅₀ approached 0.3, and then IPTG was added to a final concentration of 1 mM, and shake culturing was continued for another 1-3 hours to induce expression of the desired proteins.

One-step affinity chromatography, and when necessary also ion exchange and gel filtration chromatography, were used to purify the expressed proteins from the fractions obtained by disrupting the cells with enzymes and ultrasonic treatment.

### Binding of carrier protein and desired peptide

This was accomplished using KLH (keyhole limpet hemocyanin) as the carrier protein. That is, 100 µl of 15 mg/ml MBS (m-maleimidobenzoyl-N-hydroxysuccinimide ester) (dimethylformamide solution) was added to 10 mg/ml KLH (0.01 M phosphate buffer, pH 7.0) from which the low molecular impurities had been removed by dialysis, and the mixture was gently stirred at room temperature for 30 minutes to activate the KLH.

After removing the unreacted substance from the activated KLH using a PD-10 column (product of Pharmacia Co.), the desired peptide fragments (5 mg/ml of the synthetic peptides (1)-(4)) dissolved beforehand in a 6 M guanidine-HCl/0.01 M phosphate buffer (pH 7.0) were added, the pH was adjusted to 7.3, and after stirring at room temperature for 3 hours, the unreacted substances were removed by dialysis to prepare immunogen.

### Example 5: Antibody production

A 2 ml portion of the immunogen (1 mg/ml) obtained in Example 4 was mixed with an equivalent of Freund's complete adjuvant and emulsified, and then used to immunize Japanese white house rabbits (body weight: 2 kg) at 4 locations under the dorsal skin. After 2 weeks, the same amount of immunogen was mixed with an equivalent of Freund's incomplete adjuvant and emulsified, and used for booster immunization which was continued 2-3 times at 2-3 week intervals. At one week after the final immunization, blood was taken by a normal method and antiserum was obtained. The specific antibody titer of the obtained antiserum was measured based on the ELISA method described below.

For measurement of the serum antibody titer by the ELISA method, each the aforementioned synthetic peptides (1) to (4) was diluted in a carbonate buffer (pH 9.6) to 30 µg/ml and dispensed into a 96-well multiplate (product of Sumitomo Bakelite Co.) at 100 µl/well and left overnight at 4°C for adsorption. After washing with a PBST buffer (phosphate buffer (pH 7.4) containing 0.05% Tween20), 1% bovine serum albumin was added at 100 µl/well for blocking, after which washing was performed with the same type of PBST buffer and 100 to 10,000-fold diluted solutions of the antiserum were added at 100 µl/well and reacted at room temperature for 2 hours.

After washing with PBST, a 1000-fold diluted solution of alkaline phosphate-labelled anti-rabbit serum (goat) was added at 100 µl/well and reacted at room temperature for 2 hours. After the reaction, washing was performed with the same type of PBST buffer and then a solution of the substrate sodium p-nitrophenylphosphate dissolved at 1 mg/ml in a diethanolamine buffer (pH 9.8) was added at 100 µl/well for coloration at room temperature, and this was followed by colorimetry at 405 nm.

The results confirmed an increase in the specific antibodies against the synthetic peptides of interest.

### Example 6: Inhibition of Porphyromonas bacteria adhesion by antibodies

The adhesion inhibition test for *P*. *gingivalis* was carried out based on the following method. Five milligrams of hydroxyapatite (HA) granules of size 100-150 µm were placed in a 96-well multiplate, sterilized bland saliva was added and allowed to act at room temperature for 2 hours, and when the saliva components had been adsorbed onto the HA surface it was then washed with a KCl buffer (pH 6.0) and used as the adsorbed hydroxyapatite (S-HA).

*P*. *gingivalis* ATCC33277 (10⁷ cells) labelled with ³H-thymidine were added to the above-mentioned S-HA and reacted at room temperature for one hour for adhesion onto the S-HA. After completion of the reaction, washing was performed with the same type of buffer, and the cells adhering to the S-HA were counted with a liquid scintillation counter to determine the degree of adhesion of *P*. *gingivalis.*

The adhesion-inhibiting effects on the peptides of interest were determined by adding the test antibodies (antibodies against peptides (1) to (4)) to the experimental system described above, and the adhesion inhibition rates are shown in Table 2 as percentages with respect to the control.

**Table 2**

| Adhesion-inhibiting effect of antibodies on *P*. *gingivalis* | |
|---|---|
| Peptide antigen | Adhesion inhibition rate (%) |
| (1) | 72.1 |
| (2) | 33.6 |
| (3) | 53.6 |
| (4) | 89.9 |

The results shown in Table 2 indicate that the antibodies against peptides (1) to (4) significantly inhibit adhesion of *Porphyromonas gingivalis* to S-HA, thus confirming the usefulness of these antibodies.

### Example 7: Formula for periodontal disease prophylactic

| (1) Dentifrice | |
|---|---|
| dibasic calcium phosphate·2H₂O | 50.0% |
| sorbit | 10.0% |
| glycerin | 10.0% |
| carrageenan | 1.0% |
| sodium lauryl sulfate | 1.0% |
| aromatics | 1.0% |
| saccharin | 0.1% |
| ethanol | 2.0% |
| triclosan | 0.05% |
| anti-fimbria fragment goat milk antibody | 0.2% |
| water | remainder |
| | 100.0% |

| (2) Dentifrice | |
|---|---|
| silicic anhydride | 30.0% |
| glycerin | 30.0% |
| sorbit | 20.0% |
| carboxymethyl cellulose | 1.0% |
| sodium lauryl sulfate | 1.2% |
| aromatics | 1.0% |
| saccharin | 0.1% |
| ethanol | 2.0% |
| tranexamic acid | 0.05% |
| anti-fimbria fragment cow milk antibody | 0.1% |
| water | remainder |
| | 100.0% |

| (3) Dentifrice | |
|---|---|
| aluminum hydroxide | 45.0% |
| sorbit | 20.0% |
| carrageenan | 0.5% |
| carboxymethyl cellulose | 1.0% |
| lauryl diethanolamide | 1.0% |
| sucrose monolaurate | 2.0% |
| aromatics | 1.0% |
| saccharin | 0.1% |
| anti-fimbria fragment sheep serum antibody | 0.2% |
| water | remainder |
| | 100.0% |

| (4) Dentifrice | |
|---|---|
| dibasic calcium phosphate·2H₂O | 45.0% |
| carboxymethyl cellulose | 1.0% |
| carrageenan | 0.5% |
| sorbit | 35.0% |
| propylene glycol | 3.0% |
| N-lauroylmethyltaurine sodium | 0.5% |
| gelatin | 1.0% |
| ethyl peroxybenzoate | 0.2% |
| saccharin sodium | 0.1% |
| aromatics | 1.1% |
| magnesium ascorbate phosphate ester | 0.5% |
| anti-fimbria fragment hen egg antibody | 0.5% |
| water | remainder |
| | 100.0% |

| (5) Dentifrice | |
|---|---|
| Aluminum hydroxide | 40.0% |
| carboxymethyl cellulose | 1.0% |
| carrageenan | 0.5% |
| sorbit | 30.0% |
| propylene glycol | 3.0% |
| N-myristylmethyltaurine sodium | 0.5% |
| peptide | 1.0% |
| methyl peroxybenzoate | 0.2% |
| saccharin sodium | 0.1% |
| aromatics | 1.1% |
| cetyl pyridinium chloride | 0.5% |
| anti-fimbria fragment horse serum antibody | 0.5% |
| water | remainder |
| | 100.0% |

| (6) Mouthwash | |
|---|---|
| ethanol | 20.0% |
| aromatics | 1.0% |
| saccharin | 0.05% |
| lauryl diethanolamide | 0.3% |
| chlorhexidine gluconate | 0.01% |
| anti-fimbria fragment cow milk antibody | 0.1% |
| water | remainder |
| | 100.0% |

| (7) Mouthwash | |
|---|---|
| sorbit | 10.0% |
| ethanol | 20.0% |
| N-palmitoylmethyltaurine sodium | 0.5% |
| POE (20) sorbitan monooleate | 1.0% |
| collagen | 0.5% |
| methyl peroxybenzoate | 0.1% |
| saccharin sodium | 0.1% |
| aromatics | 0.5% |
| anti-fimbria fragment hen egg antibody | 0.4% |
| water | remainder |
| | 100.0% |

| (8) Tablets | |
|---|---|
| gum Arabic | 6.0% |
| glucose | 72.0% |
| gelatin | 3.0% |
| aromatics | 0.2% |
| 1-menthol | 0.1% |
| spearmint oil | 0.1% |
| sodium ascorbate | 0.1% |
| anti-fimbria fragment sheep milk antibody | 0.1% |
| water | remainder |
| | 100.0% |

| (9) Gum | |
|---|---|
| gum base | 43.9% |
| calcium carbonate | 2.0% |
| starch syrup | 15.0% |
| sugar | 30.0% |
| sucrose palmitate | 1.0% |
| fructose | 4.0% |
| aldose | 3.0% |
| aromatics | 1.0% |
| anti-fimbria fragment hen egg antibody | 0.1% |
| | 100.0% |

| (10) Ice cream | |
|---|---|
| cream (50% nonfat) | 16.84% |
| sugar-free nonfat condensed milk | 24.24% |
| sugar | 11.25% |
| corn syrup | 4.65% |
| stabilizers | 0.35% |
| anti-fimbria fragment antibody-containing cow milk | 37.67% |
| anti-fimbria fragment antibody-containing egg yolk | 5.00% |
| | 100.0% |

### INDUSTRIAL APPLICABILITY

When sequence portions of nucleic acids coding for fimbrillin proteins of *Porphyromonas gingivalis* cloned according to the present invention which are specific to the individual strains are used as probes, those individual strains may be separately detected, and when portions with high homology among the individual strains are used as probes, one or a few such probes may be used to detect microorganisms of the species *Porphyromonas gingivalis*. Likewise, individual strains may be separately detected by using antibodies against portions of the amino acid sequences of the fimbrillin proteins which are specific to the individual strains, and microorganisms of *Porphyromonas gingivalis* bacteria may be detected by using one or a few different antibodies against amino acid sequences with high homology among the different strains; furthermore, one or a few different antibodies may be used to inhibit adhesion of *Porphyromonas gingivalis* bacteria to teeth.

SEQUENCE LISTING
SEQ ID NO: 1
Sequence Length: 1309
Sequence Type: Nucleic acid
Strandedness: Double strand
Molecular Type: genomic DNA
Original Source
Organism: Porphyromonas gingivalis
Strain: 381
Feature
DNA containing fim A gene
216 - 218 Start codon
1257 - 1259 Stop codon
Sequence:
AGCACAACAT AATCTGAACG AACTGCGACG CTATATGCAA GACAATCTCT AAATGGGAAA 60
AGATTAGATT TTTAGAAAAC AATATTCACT TTTAAAACAA AAACGAGATG AAAAAAACAA 120
AGTTTTTCTT GTTGGGACTT GCTGCTCTTG CTATGACAGC TTGTAACAAA GACAACGAGG 180
CAGAACCCGT TACAGAAGGT AATGCCACCA TCAGCGTGGT ATTGAAGACC AGCAATTCGA 240
ATCGTGCTTT TGGAGTTGGC GATGACGAAT CAAAGGTGGC TAAGTTGACC GTAATGGTTT 300
ATAATGGAGA ACAGCAGGAA GCCATCAAAT CAGCCGAAAA TGCGACTAAG GTTGAAGACA 360
TCAAATGTAG TGCAGGCCAA CGTACGCTGG TCGTAATGGC CAATACGGGT GCAATGGAAC 420
TGGTTGGCAA GACTCTTGCA GAGGTAAAAG CATTGACAAC TGAACTGACT GCAGAAAACC 480
AAGAGGCTGC AGGGTTGATC ATGACAGCAG AGCCAAAAAC AATCGTTTTG AAGGCAGGCA 540
AGAACTACAT TGGATACAGT GGAACCGGAG AGGGTAATCA CATTGAGAAT GATCCTCTTA 600
AGATCAAGCG TGTTCATGCT CGCATGGCTT TCACCGAAAT TAAAGTGCAA ATGAGCGCAG 660
CCTACGATAA CATTTACACA TTCGTCCCTG AAAAGATTTA TGGTCTCATT GCAAAGAAGC 720
AATCTAATTT GTTCGGGGCA ACACTCGTAA ATGCAGACGC TAATTATCTG ACAGGTTCTT 780
TGACCACATT TAACGGTGCT TACACACCTG CCAACTATGC CAATGTGCCT TGGCTGAGCC 840
GTAATTACGT TGCACCTGCC GCCGATGCTC CTCAGGGTTT CTACGTATTA GAAAATGACT 900
ACTCAGCTAA CGGTGGAACT ATTCATCCGA CAATCCTGTG TGTTTATGGC AAACTTCAGA 960
AAAACGGAGC CGACTTGGCG GGAGCCGATT TAGCAGCTGC TCAGGCCGCC AATTGGGTGG 1020
ATGCAGAAGG CAAGACCTAT TACCCTGTAT TGGTAAACTT CAACAGCAAC AACTATACTT 1080
ATGACAGCAA TTATACGCCT AAGAATAAAA TTGAGCGTAA CCATAAGTAT GATATTAAGT 1140
TGACAATTAC AGGCCCCGGA ACGAATAACC CAGAGAATCC TATCACAGAG TCTGCTCACT 1200
TGAATGTACA GTGCACTGTA GCTGAGTGGG TTCTCGTTGG TCAGAATGCT ACTTGGTAAT 1260
CGACCCGTCA AACGACTAAA AAACTTTCAT AGTTTGTCTA TATCGGAAT 1309
SEQ ID NO: 2
Sequence Length: 1309
Sequence Type: Nucleic acid
Strandedness: Double strand

Molecular Type: genomic DNA
Original Source
Organism: Porphyromonas gingivalis
Strain: ATCC33227
Feature
DNA containing fim A gene
216 - 218 Start codon
1257 - 1259 Stop codon
Sequence:
AGCACAACAT AATCTGAACG AACTGCGACG CTATATGCAA GACAATCTCT AAATGGGAAA 60
AGATTAGATT TTTAGAAAAC AATATTCACT TTTAAAACAA AAACGAGATG AAAAAAACAA 120
AGTTTTTCTT GTTGGGACTT GCTGCTCTTG CTATGACAGC TTGTAACAAA GACAACGAGG 180
CAGAACCCGT TACAGAAGGT AATGCCACCA TCAGCGTGGT ATTGAAGACC AGCAATTCGA 240
ATCGTGCTTT TGGAGTTGGC GATGACGAAT CAAAGGTGGC TAAGTTGACC GTAATGGTTT 300
ATAATGGAGA ACAGCAGGAA GCCATCAAAT CAGCCGAAAA TGCGACTAAG GTTGAAGACA 360
TCAAATGTAG TGCAGGCCAA CGTACGCTGG TCGTAATGGC CAATACGGGT GCAATGGAAC 420
TGGTTGGCAA GACTCTTGCA GAGGTAAAAG CATTGACAAC TGAACTGACT GCAGAAAACC 480
AAGAGGCTGC AGGGTTGATC ATGACAGCAG AGCCAAAAAC AATCGTTTTG AAGGCAGGCA 540
AGAACTACAT TGGATACAGT GGAACCGGAG AGGGTAATCA CATTGAGAAT GATCCTCTTA 600
AGATCAAGCG TGTTCATGCT CGCATGGCTT TCACCGAAAT TAAAGTGCAA ATGAGCGCAG 660
CCTACGATAA CATTTACACA TTCGTCCCTG AAAAGATTTA TGGTCTCATT GCAAAGAAGC 720
AATCTAATTT GTTCGGGGCA ACACTCGTAA ATGCAGACGC TAATTATCTG ACAGGTTCTT 780
TGACCACATT TAACGGTGCT TACACACCTG CCAACTATGC CAATGTGCCT TGGCTGAGCC 840
GTAATTACGT TGCACCTGCC GCCGATGCTC CTCAGGGTTT CTACGTATTA GAAAATGACT 900
ACTCAGCTAA CGGTGGAACT ATTCATCCGA CAATCCTGTG TGTTTATGGC AAACTTCAGA 960
AAAACGGAGC CGACTTGGCG GGAGCCGATT TAGCAGCTGC TCAGGCCGCC AATTGGGTGG 1020
ATGCAGAAGG CAAGACCTAT TACCCTGTAT TGGTAAACTT CAACAGCAAC AACTATACTT 1080
ATGACAGCAA TTATACGCCT AAGAATAAAA TTGAGCGTAA CCATAAGTAT GATATTAAGT 1140
TGACAATTAC AGGCCCCGGA ACGAATAACC CAGAGAATCC TATCACAGAG TCTGCTCACT 1200
TGAATGTACA GTGCACTGTA GCTGAGTGGG TTCTCGTTGG TCAGAATGCT ACTTGGTAAT 1260
CGACCCGTCA AACGACTAAA AAACTTTCAT AGTTTGTCTA TATCGGAAT 1309
SEQ ID NO: 3
Sequence Length: 1309
Sequence Type: Nucleic acid
Strandedness: Double strand
Molecular Type: genomic DNA
Original Source
Organism: Porphyromonas gingivalis
Strain: BH18/10
Feature

DNA containing fim A gene
216 - 218 Start codon
1257 - 1259 Stop codon
Sequence:
AGCACAACAT AATCTGAACG AACTGCGACG CTATATGCAA GACAATCTCT AAATGGGAAA 60
AGATTAGATT TTTAGAAAAC AATATTCACT TTTAAAACAA AAACGAGATG AAAAAAACAA 120
AGTTTTTCTT GTTGGGACTT GCTGCTCTTG CTATGACAGC TTGTAACAAA GACAACGAGG 180
CAGAACCCGT TACAGAAGGT AATGCCACCA TCAGCGTGGT ATTGAAGACC AGCAATTCGA 240
ATCGTGCTTT TGGAGTTGGC GATGACGAAT CAAAGGTGGC TAAGTTGACC GTAATGGTTT 300
ATAATGGAGA ACAGCAGGAA GCCATCAAAT CAGCCGAAAA TGCGACTAAG GTTGAAGACA 360
TCAAATGTAG TGCAGGCCAA CGTACGCTGG TCGTAATGGC CAATACGGGT GCAATGGAAC 420
TGGTTGGCAA GACTCTTGCA GAGGTAAAAG CATTGACAAC TGAACTGACT GCAGAAAACC 480
AAGAGGCTGC AGGGTTGATC ATGACAGCAG AGCCAAAAAC AATCGTTTTG AAGGCAGGCA 540
AGAACTACAT TGGATACAGT GGAACCGGAG AGGGTAATCA CATTGAGAAT GATCCTCTTA 600
AGATCAAGCG TGTTCATGCT CGCATGGCTT TCACCGAAAT TAAAGTGCAA ATGAGCGCAG 660
CCTACGATAA CATTTACACA TTCGTCCCTG AAAAGATTTA TGGTCTCATT GCAAAGAAGC 720
AATCTAATTT GTTCGGGGCA ACACTCGTAA ATGCAGACGC TAATTATCTG ACAGGTTCTT 780
TGACCACATT TAACGGTGCT TACACACCTG CCAACTATGC CAATGTGCCT TGGCTGAGCC 840
GTAATTGCGT TGCACCTGCC GCCGATGCTC CTCAGGGTTT CTACGTATTA GAAAATGACT 900
ACTCAGCTAA CGGTGGAACT ATTCATCCGA CAATCCTGTG TGTTTATGGC AAACTTCAGA 960
AAAACGGAGC CGACTTGGCG GGAGCCGATT TAGCAGCTGC TCAGGCCGCC AATTGGGTGG 1020
ATGCAGAAGG CAAGACCTAT TACCCTGTAT TGGTAAACTT CAACAGCAAC AACTATACTT 1080
ATGACAGCAA TTATACGCCT AAGAATAAAA TTGAGCGTAA CCATAAGTAT GATATTAAGT 1140
TGACAATTAC AGGCCCCGGA ACGAATAACC CAGAGAATCC TATCACAGAG TCTGCTCACT 1200
TGAATGTACA GTGCACTGTA GCTGAGTGGG TTCTCGTTGG TCAGAATGCT ACTTGGTAAT 1260
CGACCCGTCA AACGACTAAA AAACTTTCAT AGTTTGTCTA TATCGGAAT 1309
SEQ ID NO: 4
Sequence Length: 1306
Sequence Type: Nucleic acid
Strandedness: Double strand
Molecular Type: genomic DNA
Original Source
Organism: Porphyromonas gingivalis
Strain: HW24D-1
Feature
DNA containing fim A gene
211 - 213 Start codon
1255 - 1257 Stop codon
Sequence:
AGCACAACAC AATCTGAACG AACTACGACG CTATATGCAA GACAATCTCT AAATGGGAAA 60

AGATTCTTAG AAAACAATAT TCACTTTTAA AACAAAAACG AGATGAAAAA AACAAAGTTT 120
TTCTTGTTGG GACTTGCTGC TCTTGCTATG ACAGCTTGTA ACAAAGACAA CGAGGCAGAA 180
CCCGTTACAG AAGGTAATGC TACCATCAGC GTGGTATTGA AGACCAGCAA TCCGAATCGT 240
GCTTTTGGAG AAGACGAATC AAAGGTGGCT AAGTTGACCG TAATGGTTTA TAATGGAGAA 300
CAGCAGGAAG CCATCAAATC AGCCGAAAAT GCGACTAAGG TTGAAGACAT CAAATGTAGT 360
GCAGGCCAAC GTACGCTGGT CGTAATGGCC AATACGGGTG AAATGAAATT GGCTGGCAAG 420
ACTCTTGCAG AGGTAAAAGC ATTGACAACT GAACTGACTG CAGAAAACCA AGAGGCTGCA 480
GGGTTGATCA TGACGGCAGA GCCTGTTGAG GTAACACTTG TCGCCGGCAA TAACTATTAT 540
GGTTATGATG GATCTCAGGG AGGTAATCAG ATTTCGCAAG ATACTCCTCT TGAAATCAAA 600
CGTGTTCATG CTCGCATGGC TTTCACCGAA ATTAAAGTGC AGATGAGTCC GTCTTATGTT 660
AACAAATACA ATTTTGCCCC CGAAAACATC TATGCACTTG TGGCTAAAAA GGAGTCTAAT 720
CTATTCGGTG CTTCATTGGC AAATAGTGAT GATGCTTATT TGACTGGTTC TTTGACGAAT 780
TTCAACGGTG CTTATTCCCC TGCAAACTAT ACTCATGTTG ACTGGTTGGG AAGAGACTAC 840
ACAGAGCCTT CCAATAATGC TCCACAAGGT TTCTATGTTT TGGAGAGCAC ATACGCTCAG 900
AATGCAGGTC TACGTCCTAC TATTCTATGT GTAAAAGGCA AGCTGACAAA GCATGATGGT 960
ACTCCTTTGA GTTCTGAGGA AATGACAGCT GCATTCAATG CCGGCTGGAT TGTTGCAGAC 1020
AATAATCCTA CGACCTATTA CCCTGTATTG GTAAACTTCA ACAGCAACAA CTATACTTAT 1080
GACAATGGTT ATACGCCTAA GAATAAAATT GAGCGTAACC ATAAGTATGA TATTAAGTTG 1140
ACAATTACAG GCCCCGGAAC GAATAACCCA GAGAATCCTA TCACAGAGTC TGCTCACTTG 1200
AATGTACAGT GCACTGTAGC TGAGTGGGTT CTCGTTGGTC AGAATGCTAC TTGGTAATCG 1260
ACCCTCAAAC GACTAAAAAA CTTTCATAGT TTGTCTATAT CGGAAT 1306
SEQ ID NO: 5
Sequence Length: 1306
Sequence Type: Nucleic acid
Strandedness: Double strand
Molecular Type: genomic DNA
Original Source
Organism: Porphyromonas gingivalis
Strain: OMZ314
Feature
DNA containing fim A gene
211 - 213 Start codon
1252 - 1254 Stop codon
Sequence:
AGCACAACAT AATCTGAACG AACTACGACG CTATATGCAA GACAATCTCT AAATGGGAAA 60
AGATTCTTAG AAAACAATAT TCACTTTTAA AACAAAAACG AGATGAAAAA AACAAAGTTT 120
TTCTTGTTGG GACTTGCTGC TCTTGCTATG ACAGCTTGTA ACAAAGACAA CGAGGCAGAA 180
CCCGTTACAG AAGGTAATGC TACCATCAGC GTGGTATTGA AGACCAGCAA TCCGAATCGT 240
GCTTTTGGAG AAGACGAATC AAAGGTGGCT AAGTTGACCG TAATGGTTTA TAATGGAGAA 300
CAGCAGGAAG CCATCAAATC AGCCGAAAAT GCGACTAAGG TTGAAGACAT CAAATGTAGT 360
GCAGGCCAAC GTACGCTGGT CGTAATGGCC AATACGGGTG AAATGAAATT GGCTGGCAAG 420

ACTCTTGCAG AGGTAAAAGC ATTGACAACT GAACTGACTG CAGAAAACCA AGAGGCTGCA 480
GGGTTGATCA TGACAGCAGA GCCTGTTGAG GTAACACTTG TCGCCGGCAA TAACTATTAT 540
GGTTATGATG GATCTCAGGG AGGTAATCAG ATTTCGCAAG ATACTCCTCT TGAAATCAAA 600
CGTGTTCATG CTCGCATGGC TTTCACCGAA ATTAAAGTGC AGATGAGTCC GTCTTATGTT 660
AACAAATACA ATTTTGCCCC CGAAAACATC TATGCACTTG TGGCTAAAAA GGAGTCTAAT 720
CTATTCGGTG CTTCATTGGC AAATAGTGAT GATGCTTATT TGACTGGTTC TTTGACGAAT 780
TTCAACGGTG CTTATTCCCC TGCAAACTAT ACTCATGTTG ACTGGTTGGG AAGAGACTAC 840
ACAGAGCCTT CCAATAATGC TCCACAAGGT TTCTATGTTT TGGAGAGCAC ATACGCTCAG 900
AATGCAGGTC TACGTCCTAC TATTCTATGT GTAAAAGGCA AGCTGACAAA GCATGATGGT 960
ACTCCTTTGA GTTCTGAGGA AATGACAGCT GCATTCAATG CCGGCTGGAT TGTTGCAGAC 1020
AATAATCCTA CGACCTATTA CCCTGTATTG GTAAACTTCA ACAGCAACAA CTATACTTAT 1080
GACAATGGTT ATACGCCTAA GAATAAAATT GAGCGTAACC ATAAGTATGA TATTAAGTTG 1140
ACAATTACAG GCCCCGGAAC GAATAACCCA GAGAATCCTA TCACAGAGTC TGCTCACTTG 1200
AATGTACAGT GCACTGTAGC TGAGTGGGTT CTCGTTGGTC AGAATGCTAC TTGATAATCG 1260
GCCCTCAAAC GACTAAAAAA CTTTCATAGT TTGTCTATAT CGGAAT 1306
SEQ ID NO: 6
Sequence Length: 1311
Sequence Type: Nucleic acid
Strandedness: Double strand
Molecular Type: genomic DNA
Original Source
Organism: Porphyromonas gingivalis
Strain: OMZ409
Feature
DNA containing fim A gene
216 - 218 Start codon
1260 - 1262 Stop codon
Sequence:
AGCACAACAT AATCTGAACG AACTACGACG CTATATGCAA GACAATCTCT AAATGGGAAA 60
AGATTAGATT CTTAGAAAAC AATATTCACT TTTAAAACAA AAACGAGATG AAAAAAACAA 120
AGTTTTTCTT GTTGGGACTT GCTGCTCTTG CTATGACAGC TTGTAACAAA GACAACGAGG 180
CAGAACCCGT TACAGAAGGT AATGCTACCA TCAGCGTGGT TTTGAAGACC AGCAATCCGA 240
ATCGTGCTTT TGGAGAAGAC GAATCAAAGG TGGCTAAGTT GACCGTAATG GTTTATAATG 300
GAGAACAGCA GGAAGCCATC AAATCAGCCG AAAATGCGAC TAAGGTTGAA GACATCAAAT 360
GTAGTGCAGG CCAACGTACG CTGGTCGTAA TGGCCAATAC GGGTGCAATG GAACTGGTTG 420
GCAAGACTCT TGCAGAGGTA AAAGCATTGA CAACTGAACT GACTGCAGAA AACCAAGAGG 480
CTACAGGTTT GATCATGACA GCAGAGCCTG TTGACGTAAC ACTTGTCGCC GGCAATAACT 540
ATTATGGTTA TGATGGATCT CAGGGAGGTA ATCAGATTTC GCAGGATACT CCTCTTGAAA 600
TCAAACGTGT TCATGCTCGC ATGGCTTTCA CCGAAATTAA AGTGCAGATG AGTCCGTCTT 660
ATGTTAACAA ATACAATTTT GCCCCCGAAA ACATCTATGC ACTTGTGGCT AAAAAGGAGT 720
CTAATCTATT CGGTGCTTCA TTGGCAAATA GTGATGATGC TTATTTGACT GGTTCTTTGA 780

CGAATTTCAA CGGTGCTTAT TCCCCTGCAA ACTATACTCA TGTTGACTGG TTGGGAAGAG 840
ACTACACAGA GCCTTCCAAT AATGCTCCAC AAGGTTTCTA TGTTTTGGAG AGCACATACG 900
CTCAGAATGC AGGTCTACGT CCTACTATTC TATGTGTAAA AGGCAAGCTG ACAAAGCATG 960
ATGGTACTCC TTTGAGTTCT GAGGAAATGA CAGCTGCATT CAATGCCGGC TGGATTGTTG 1020
CAGACAATAA TCCTACGACC TATTACCCTG TATTGGTAAA CTTCAACAGC AACAACTATA 1080
CTTATGACAA TGGTTATACG CCTAAGAATA AAATTGAGCG TAACCATAAG TATGATATTA 1140
AGTTGACAAT TACAGGCCCC GGAACGAATA ACCCAGAGAA TCCTATCACA GAGTCTGCTC 1200
ACTTGAATGT ACAGTGCACT GTAGCTGAGT GGGTTCTCGT TGGTCAGAAT GCTACTTGGT 1260
AATCGACCCT CAAACGACTA AAAAACTTTC ATAGTTTGTC TATATCGGAA T 1311
SEQ ID NO: 7
Sequence Length: 1318
Sequence Type: Nucleic acid
Strandedness: Double strand
Molecular Type: genomic DNA
Original Source
Organism: Porphyromonas gingivalis
Strain: ATCC49417
Feature
DNA containing fim A gene
216 - 218 Start codon
1266 - 1268 Stop codon
Sequence:
AGCACAACAC AATCTGAACG AACTACGACG CTATATGCAA GACAATCTCT AAATGGGAAA 60
AGATTAGATT CTTAGAAAAC AATATTCACT TTTAAAACAA AAACGAGATG AAAAAAACAA 120
AGTTTTTCTT GTTGGGACTT GCTGCTCTTG CTATGACAGC TTGTAACAAA GACAACGAGG 180
CAGAACCTGT TACAGAAGGT AATGCTACCA TCAGCGTGGT ATTGAAGACC AGCAATCCGA 240
ATCGTGCTTT TGGAAATGCG GGAGACGAAG CAAAAGTGGC TAAGTTGACC GTAATGGTTT 300
ACAAGGGTGA ACAGCAGGAA GCCATCAAAT CAGCCGAAAA TGCGACTAAG GTTGAAAACA 360
TCAAATGTAG TGCAGGCCAA CGTACGCTGG TCGTAATGGC CAATACGGGT GGAATGGAAT 420
TGGCTGGCAA GACTCTTGCA GAGGTAAAAG CATTGACAAC TGAACTGACT GAAGGAAACC 480
AAGAGGCTGC AGGGTTGATC ATGACAGCAG AGCCTGTTGA GGTAACACTT GTCGCCGGCA 540
ATAACTATTA TGGTTATGAT GGATCTCAGG GAGGTAATCA GATTTCGCAA GATACTCCTC 600
TTGAAATCAA ACGTGTTCGT GCCCGTATTG CGTTCACCAA GATTGAAGTG ACGATGAGCC 660
AGTCTTATGC GAACAAATAC AATTTTGCCC CCGAAAACAT CTATGCACTT GTGGCTAAAA 720
AGAAGTCTAA TCTATTCGGT GCTTCATTGG CAAATAATGA TGATGCTTAT TTGACTGGTT 780
CTTTGACGAC TTTCAACGGA GCTTATACCC CTGCAAACTA TACTCATGTC GACTGGTTGG 840
GAAGAGACTT CACAGAGCCT TCCAATAATG CTCCACAAGG TTTCTATGTT TTGGAGAGCA 900
CATACGCTCA GAATGCAGGT CTACGTCCTA CTATTCTATG TATAAAAGGC AAGCTGACAA 960
AGCATGATGG TACTCCTTTG AGTTCTGAGG AAATGACAGC TGCATTCAAT GCCGGCTGGA 1020
TTGTTGCAAA CAATGATCCT ACGACCTATT ATCCTGTATT AGTGAACTTT GAGAGCAATA 1080
ATTACACCTA CACAGGTGAG GCTGTTGAGA AAGGAAAAAT CGTTCGTAAC CATAAATTCG 1140

ACATCAACCT GACGATCACC GGTCCTGGTA CGAATAATCC TGAAAACCCC ATTACTGAGT 1200
CTGCTAACCT CAACGTTAAT TGTGTGGTTG CTGCCTGGAA AGGTGTTGTA CAAAATGTTA 1260
TTTGGTAATC GACCCGTCAA ACGACTAAAA AACTTTCATA GTTTGTCTAT ATCGGAAT 1318
SEQ ID NO: 8
Sequence Length: 1314
Sequence Type: Nucleic acid
Strandedness: Double strand
Molecular Type: genomic DNA
Original Source
Organism: Porphyromonas gingivalis
Strain: 6/26
Feature
DNA containing fim A gene
211 - 213 Start codon
1270 - 1272 Stop codon
Sequence:
AGCACAACAT AATCTGAACG AACTGCGACG CTATATGCAA GACAATCTCT AAATGGAAAA 60
AGATTCTTAG AAAACAATAT TCACTTTTAA AACAAAAACG AGATGAAAAA AACAAAGTTT 120
TTCTTGTTGG GACTTGCTGC TCTTGCTATG ACAGCTTGTA ACAAAGACAA CGAGGCAGAA 180
CCCGTTACAG AAAGTAATGC TACCATCAGC GTGGTATTGA AGACCAGCAA TCCGAATCGT 240
GCTTTTGGAA ATGCGGGAGA CGAAGCAAAA GTGGCTAAAC TGACTGTAAT GGTTTACAAG 300
GGTGAGCAGC AGGAAGCCAT CAAATCAGTC GAAAATGCAA TTAAGGTTGA AAACATCAAA 360
TGTGGTGCAG GCCAACGTAC GCTGGTTGTA ATGGCCAATA CGGGTGGAAT GGAATTGGCT 420
GGCAAAACTC TTGCAGAGGT AAAAGCATTG ACAACTGAAC TGACTGAAGG AAACCAAGAG 480
GCTGCAGGGT TGATCATGAC AGCAGAGCCT GTTGAGGTAA CACTTGTCGC CGGCAATAAC 540
TATTATGGTT ATGATGGATC TCAGGGAGGT AATCAGATTT CGCAAGGTAC TCCTCTTGAA 600
ATCAAACGTG TTCATGCCCG TATTGCGTTC ACCAAGATTG AAGTGACGAT GAGCCAGTCT 660
TATGCGAACA AATACAATTT TGCCCCCGAA AACATCTATG CACTTGTGGC TAAAAAGAAG 720
TCTAATCTAT TCGGTGCTTC ATTGGCAAAT AGTGATGATG CTTATTTGAC TGGTTCTTTG 780
ACGACTTTCA ACGGTGCTTA TTCCCCTGCA AACTATACTC ATGTTGACTG GTTGGGAAGA 840
GACTACACAG AAATAGGAGC CGCTACTGTT AATACTCCGA AGGGATTCTA TGTCTTGGAG 900
AGCACATACG CTCAGAATGC AGGTCTACGT CCTACTATTC TATGTGTAAA AGGCAAGCTG 960
ACAAAGCATG ATGGTACAGC TTTGAGTTCT GAGGAAATGA CAGCTGCATT CAATGCCGGC 1020
TGGATTGTTG CAAACAATGA TCCTACGACC TATTATCCTG TATTAGTGAA CTTTGAGAGC 1080
AATAATTACA CCTACACAGG TGAGGCTGTT GAGAAAGGAA AAATCGTTCG TAACCATAAG 1140
TTCGACATCA ACCTGACGAT CACCGGTCCT GGCACGAATA ATCCTGAAAA CCCCATTACT 1200
GAGTCTGCTA ACCTCAACGT TAATTGTGTG GTTGCTGCAT GGAAAGGTGT TGTACAAAAT 1260
GTTATTTGGT AATCAGCTCA TCAAAAAACT TTCATAGTTT GTCTATATCG GAAT 1314
SEQ ID NO: 9
Sequence Length: 1319
Sequence Type: Nucleic acid

Strandedness: Double strand
Molecular Type: genomic DNA
Original Source
Organism: Porphyromonas gingivalis
Strain: HG564
Feature
DNA containing fim A gene
187 - 189 Start codon
1267 - 1269 Stop codon
Sequence:
AGCACAACAC AATCTGAACG AACTGCGACG CTATATGCAA GACAATCTCT AAATGGAAAA 60
AGATTCTTAG AAAACAATAT TCACTTTTAA AACAAAAACG AGATGAAAAA AACAAAGTTT 120
TTCTTGTTGG GACTTGCTGC TCTTGCTATG ACAGCTTGTA ACAAAGACAA CGAGGCAGAA 180
CCCATTGTGG AAACTGACGC TACTGTTAGT TTCATAATTA AGAGCGGAGA GGGGCGTGCT 240
GTAGGCGATG GCCTTGCAGA TGCCAAGATC ACAAAACTCA CCGCCATGGT CTATGCAGGT 300
CAAATTCAAG AAGGGATTAA GACAGTGGAA GAGGCCGACG GAGTTCTTAA AGTAGAAGGA 360
ATTCCGTGTA AATCAGGAGC CAACCGTGTC CTCGTCGTTG TAGCTAATCA CAATTATGAG 420
CTTACCGGTA AAAGTTTGAA TGAGGTTGAG GCCTTGACGA CTTCTTTGAC AGCTGAAAAC 480
CAAAATGCCA AAAACTTGAT CATGACAGGT AAGTCAGCAG CTTTTACAAT CAAGCCGGGC 540
TCCAACCACT ATGGCTATCC TGATGGGACT ACATCCGACA ACCTTGTTTC TGCTGGAACT 600
CCTCTTGCCG TTACTCGCGT GCATGCCGGT ATCTCATTCG CAGGAGTAGA GGTAAATATG 660
GCTACACAGT ATCAAAACTA CTACTCTTTT AACCCAGCTG ACGCTAAAAT CGCAGCCCTT 720
GTCGCAAAGA AAGATTCTAA GATTTTCGGC AATTCTTTGG TCTCAAACAC TAATGCATAT 780
TTGTATGGAG TCCAAACGCC TGCCGGTCTT TACACTCCGG ATGCTGCAGG AGAAACATAC 840
GAATTGGAGG CGTCTTTGAA TACGAATTAT GCTGTAGGTG CCGGCTTCTA TGTGCTGGAA 900
AGTAAATATG ATGCAAGCAA CGAGCTTCGT CCGACGATCC TTTGTATCTA TGGAAAGCTG 960
CTCGATAAGG ACGGCAACCC TCTCACGGAA CCAGCCTTGA CGGATGCTAT AAATGCCGGA 1020
TTCTGCGACG GAGATGGCAC GACTTACTAT CCGGTATTGG TGAACTATGA TGGCAATGGC 1080
TACATCTATT CAGGTGCTAT TACCCAAGGA CAAAACAAAA TCGTTCGCAA CAACCACTAC 1140
AAGATTACGC TGAACATCAC CGGCCCCGGT ACGAATACTC CTGAAAATCC TCAACCGGTA 1200
CAAGCCAACC TGAATGTTAC TTGCCAAGTT ACACCTTGGG TTGTTGTTAA TCAGGCTGCT 1260
ACTTGGTAAT CGACCCGTCA AACGACTAAA AAACTTTCAT AGTTTGTCTA TATCGGAAT 1319
SEQ ID NO: 10
Sequence Length: 347
Sequence Type: Amino acid
Topology: Linear
Molecular Type: Protein
Feature: Amino acid sequence of finbrillin of
Porphyromonas gingivalis 381
Sequence:

Met Val Leu Lys Thr Ser Asn Ser Asn Arg Ala Phe Gly Val Gly Asp
-10 -5 -1 1 5
Asp Glu Ser Lys Val Ala Lys Leu Thr Val Met Val Tyr Asn Gly Glu
10 15 20
Gln Gln Glu Ala Ile Lys Ser Ala Glu Asn Ala Thr Lys Val Glu Asp
25 30 35
Ile Lys Cys Ser Ala Gly Gln Arg Thr Leu Val Val Met Ala Asn Thr
40 45 50
Gly Ala Met Glu Leu Val Gly Lys Thr Leu Ala Glu Val Lys Ala Leu
55 60 65 70
Thr Thr Glu Leu Thr Ala Glu Asn Gln Glu Ala Ala Gly Leu Ile Met
75 80 85
Thr Ala Glu Pro Lys Thr Ile Val Leu Lys Ala Gly Lys Asn Tyr Ile
90 95 100
Gly Tyr Ser Gly Thr Gly Glu Gly Asn His Ile Glu Asn Asp Pro Leu
105 110 115
Lys Ile Lys Arg Val His Ala Arg Met Ala Phe Thr Glu Ile Lys Val
120 125 130
Gln Met Ser Ala Ala Tyr Asp Asn Ile Tyr Thr Phe Val Pro Glu Lys
135 140 145 150
Ile Tyr Gly Leu Ile Ala Lys Lys Gln Ser Asn Leu Phe Gly Ala Thr
155 160 165
Leu Val Asn Ala Asp Ala Asn Tyr Leu Thr Gly Ser Leu Thr Thr Phe
170 175 180
Asn Gly Ala Tyr Thr Pro Ala Asn Tyr Ala Asn Val Pro Trp Leu Ser
185 190 195
Arg Asn Tyr Val Ala Pro Ala Ala Asp Ala Pro Gln Gly Phe Tyr Val
200 205 210
Leu Glu Asn Asp Tyr Ser Ala Asn Gly Gly Thr Ile His Pro Thr Ile
215 220 225 230
Leu Cys Val Tyr Gly Lys Leu Gln Lys Asn Gly Ala Asp Leu Ala Gly
235 240 245
Ala Asp Leu Ala Ala Ala Gln Ala Ala Asn Trp Val Asp Ala Glu Gly
250 255 260
Lys Thr Tyr Tyr Pro Val Leu Val Asn Phe Asn Ser Asn Asn Tyr Thr
265 270 275
Tyr Asp Ser Asn Tyr Thr Pro Lys Asn Lys Ile Glu Arg Asn His Lys
280 285 290
Tyr Asp Ile Lys Leu Thr Ile Thr Gly Pro Gly Thr Asn Asn Pro Glu
295 300 305 310
Asn Pro Ile Thr Glu Ser Ala His Leu Asn Val Gln Cys Thr Val Ala
315 320 325
Glu Trp Val Leu Val Gly Gln Asn Ala Thr Trp
330 335

SEQ ID NO: 11
Sequence Length: 347
Sequence Type: Amino acid
Topology: Linear
Molecular Type: Protein
Feature: Amino acid sequence of finbrillin of
Porphyromonas gingivalis ATCC33277
Sequence:
Met Val Leu Lys Thr Ser Asn Ser Asn Arg Ala Phe Gly Val Gly Asp
-10 -5 -1 1 5
Asp Glu Ser Lys Val Ala Lys Leu Thr Val Met Val Tyr Asn Gly Glu
10 15 20
Gln Gln Glu Ala Ile Lys Ser Ala Glu Asn Ala Thr Lys Val Glu Asp
25 30 35
Ile Lys Cys Ser Ala Gly Gln Arg Thr Leu Val Val Met Ala Asn Thr
40 45 50
Gly Ala Met Glu Leu Val Gly Lys Thr Leu Ala Glu Val Lys Ala Leu
55 60 65 70
Thr Thr Glu Leu Thr Ala Glu Asn Gln Glu Ala Ala Gly Leu Ile Met
75 80 85
Thr Ala Glu Pro Lys Thr Ile Val Leu Lys Ala Gly Lys Asn Tyr Ile
90 95 100
Gly Tyr Ser Gly Thr Gly Glu Gly Asn His Ile Glu Asn Asp Pro Leu
105 110 115
Lys Ile Lys Arg Val His Ala Arg Met Ala Phe Thr Glu Ile Lys Val
120 125 130
Gln Met Ser Ala Ala Tyr Asp Asn Ile Tyr Thr Phe Val Pro Glu Lys
135 140 145 150
Ile Tyr Gly Leu Ile Ala Lys Lys Gln Ser Asn Leu Phe Gly Ala Thr
155 160 165
Leu Val Asn Ala Asp Ala Asn Tyr Leu Thr Gly Ser Leu Thr Thr Phe
170 175 180
Asn Gly Ala Tyr Thr Pro Ala Asn Tyr Ala Asn Val Pro Trp Leu Ser
185 190 195
Arg Asn Tyr Val Ala Pro Ala Ala Asp Ala Pro Gln Gly Phe Tyr Val
200 205 210
Leu Glu Asn Asp Tyr Ser Ala Asn Gly Gly Thr Ile His Pro Thr Ile
215 220 225 230
Leu Cys Val Tyr Gly Lys Leu Gln Lys Asn Gly Ala Asp Leu Ala Gly
235 240 245
Ala Asp Leu Ala Ala Ala Gln Ala Ala Asn Trp Val Asp Ala Glu Gly
250 255 260

Lys Thr Tyr Tyr Pro Val Leu Val Asn Phe Asn Ser Asn Asn Tyr Thr
265 270 275
Tyr Asp Ser Asn Tyr Thr Pro Lys Asn Lys Ile Glu Arg Asn His Lys
280 285 290
Tyr Asp Ile Lys Leu Thr Ile Thr Gly Pro Gly Thr Asn Asn Pro Glu
295 300 305 310
Asn Pro Ile Thr Glu Ser Ala His Leu Asn Val Gln Cys Thr Val Ala
315 320 325
Glu Trp Val Leu Val Gly Gln Asn Ala Thr Trp
330 335
SEQ ID NO: 12
Sequence Length: 347
Sequence Type: Amino acid
Topology: Linear
Molecular Type: Protein
Feature: Amino acid sequence of finbrillin of
Porphyromonas gingivalis BH18/10
Sequence:
Met Val Leu Lys Thr Ser Asn Ser Asn Arg Ala Phe Gly Val Gly Asp
-10 -5 -1 1 5
Asp Glu Ser Lys Val Ala Lys Leu Thr Val Met Val Tyr Asn Gly Glu
10 15 20
Gln Gln Glu Ala Ile Lys Ser Ala Glu Asn Ala Thr Lys Val Glu Asp
25 30 35
Ile Lys Cys Ser Ala Gly Gln Arg Thr Leu Val Val Met Ala Asn Thr
40 45 50
Gly Ala Met Glu Leu Val Gly Lys Thr Leu Ala Glu Val Lys Ala Leu
55 60 65 70
Thr Thr Glu Leu Thr Ala Glu Asn Gln Glu Ala Ala Gly Leu Ile Met
75 80 85
Thr Ala Glu Pro Lys Thr Ile Val Leu Lys Ala Gly Lys Asn Tyr Ile
90 95 100
Gly Tyr Ser Gly Thr Gly Glu Gly Asn His Ile Glu Asn Asp Pro Leu
105 110 115
Lys Ile Lys Arg Val His Ala Arg Met Ala Phe Thr Glu Ile Lys Val
120 125 130
Gln Met Ser Ala Ala Tyr Asp Asn Ile Tyr Thr Phe Val Pro Glu Lys
135 140 145 150
Ile Tyr Gly Leu Ile Ala Lys Lys Gln Ser Asn Leu Phe Gly Ala Thr
155 160 165
Leu Val Asn Ala Asp Ala Asn Tyr Leu Thr Gly Ser Leu Thr Thr Phe
170 175 180

Asn Gly Ala Tyr Thr Pro Ala Asn Tyr Ala Asn Val Pro Trp Leu Ser
185 190 195
Arg Asn Cys Val Ala Pro Ala Ala Asp Ala Pro Gln Gly Phe Tyr Val
200 205 210
Leu Glu Asn Asp Tyr Ser Ala Asn Gly Gly Thr Ile His Pro Thr Ile
215 220 225 230
Leu Cys Val Tyr Gly Lys Leu Gln Lys Asn Gly Ala Asp Leu Ala Gly
235 240 245
Ala Asp Leu Ala Ala Ala Gln Ala Ala Asn Trp Val Asp Ala Glu Gly
250 255 260
Lys Thr Tyr Tyr Pro Val Leu Val Asn Phe Asn Ser Asn Asn Tyr Thr
265 270 275
Tyr Asp Ser Asn Tyr Thr Pro Lys Asn Lys Ile Glu Arg Asn His Lys
280 285 290
Tyr Asp Ile Lys Leu Thr Ile Thr Gly Pro Gly Thr Asn Asn Pro Glu
295 300 305 310
Asn Pro Ile Thr Glu Ser Ala His Leu Asn Val Gln Cys Thr Val Ala
315 320 325
Glu Trp Val Leu Val Gly Gln Asn Ala Thr Trp
330 335
SEQ ID NO: 13
Sequence Length: 348
Sequence Type: Amino acid
Topology: Linear
Molecular Type: Protein
Feature: Amino acid sequence of finbrillin of
Porphyromonas gingivalis HW24D1
Sequence:
Met Val Leu Lys Thr Ser Asn Pro Asn Arg Ala Phe Gly Glu Asp Glu
-10 -5 -1 1 5
Ser Lys Val Ala Lys Leu Thr Val Met Val Tyr Asn Gly Glu Gln Gln
10 15 20
Glu Ala Ile Lys Ser Ala Glu Asn Ala Thr Lys Val Glu Asp Ile Lys
25 30 35
Cys Ser Ala Gly Gln Arg Thr Leu Val Val Met Ala Asn Thr Gly Glu
40 45 50
Met Lys Leu Ala Gly Lys Thr Leu Ala Glu Val Lys Ala Leu Thr Thr
55 60 65 70
Glu Leu Thr Ala Glu Asn Gln Glu Ala Ala Gly Leu Ile Met Thr Ala
75 80 85
Glu Pro Val Glu Val Thr Leu Val Ala Gly Asn Asn Tyr Tyr Gly Tyr
90 95 100

Asp Gly Ser Gln Gly Gly Asn Gln Ile Ser Gln Asp Thr Pro Leu Glu
105 110 115
Ile Lys Arg Val His Ala Arg Met Ala Phe Thr Glu Ile Lys Val Gln
120 125 130
Met Ser Pro Ser Tyr Val Asn Lys Tyr Asn Phe Ala Pro Glu Asn Ile
135 140 145 150
Tyr Ala Leu Val Ala Lys Lys Glu Ser Asn Leu Phe Gly Ala Ser Leu
155 160 165
Ala Asn Ser Asp Asp Ala Tyr Leu Thr Gly Ser Leu Thr Asn Phe Asn
170 175 180
Gly Ala Tyr Ser Pro Ala Asn Tyr Thr His Val Asp Trp Leu Gly Arg
185 190 195
Asp Tyr Thr Glu Pro Ser Asn Asn Ala Pro Gln Gly Phe Tyr Val Leu
200 205 210
Glu Ser Thr Tyr Ala Gln Asn Ala Gly Leu Arg Pro Thr Ile Leu Cys
215 220 225 230
Val Lys Gly Lys Leu Thr Lys His Asp Gly Thr Pro Leu Ser Ser Glu
235 240 245
Glu Met Thr Ala Ala Phe Asn Ala Gly Trp Ile Val Ala Asp Asn Asn
250 255 260
Pro Thr Thr Tyr Tyr Pro Val Leu Val Asn Phe Asn Ser Asn Asn Tyr
265 270 275
Thr Tyr Asp Asn Gly Tyr Thr Pro Lys Asn Lys Ile Glu Arg Asn His
280 285 290
Lys Tyr Asp Ile Lys Leu Thr Ile Thr Gly Pro Gly Thr Asn Asn Pro
295 300 305 310
Glu Asn Pro Ile Thr Glu Ser Ala His Leu Asn Val Gln Cys Thr Val
315 320 325
Ala Glu Trp Val Leu Val Gly Gln Asn Ala Thr Trp
330 335
SEQ ID NO: 14
Sequence Length: 347
Sequence Type: Amino acid
Topology: Linear
Molecular Type: Protein
Feature: Amino acid sequence of finbrillin of
Porphyromonas gingivalis OMZ314
Sequence:
Met Val Leu Lys Thr Ser Asn Pro Asn Arg Ala Phe Gly Glu Asp Glu
-10 -5 -1 1 5
Ser Lys Val Ala Lys Leu Thr Val Met Val Tyr Asn Gly Glu Gln Gln
10 15 20

Glu Ala Ile Lys Ser Ala Glu Asn Ala Thr Lys Val Glu Asp Ile Lys
25 30 35
Cys Ser Ala Gly Gln Arg Thr Leu Val Val Met Ala Asn Thr Gly Glu
40 45 50
Met Lys Leu Ala Gly Lys Thr Leu Ala Glu Val Lys Ala Leu Thr Thr
55 60 65 70
Glu Leu Thr Ala Glu Asn Gln Glu Ala Ala Gly Leu Ile Met Thr Ala
75 - 80 85
Glu Pro Val Glu Val Thr Leu Val Ala Gly Asn Asn Tyr Tyr Gly Tyr
90 95 100
Asp Gly Ser Gln Gly Gly Asn Gln Ile Ser Gln Asp Thr Pro Leu Glu
105 110 115
Ile Lys Arg Val His Ala Arg Met Ala Phe Thr Glu Ile Lys Val Gln
120 125 130
Met Ser Pro Ser Tyr Val Asn Lys Tyr Asn Phe Ala Pro Glu Asn Ile
135 140 145 150
Tyr Ala Leu Val Ala Lys Lys Glu Ser Asn Leu Phe Gly Ala Ser Leu
155 160 165
Ala Asn Ser Asp Asp Ala Tyr Leu Thr Gly Ser Leu Thr Asn Phe Asn
170 175 180
Gly Ala Tyr Ser Pro Ala Asn Tyr Thr His Val Asp Trp Leu Gly Arg
185 190 195
Asp Tyr Thr Glu Pro Ser Asn Asn Ala Pro Gln Gly Phe Tyr Val Leu
200 205 210
Glu Ser Thr Tyr Ala Gln Asn Ala Gly Leu Arg Pro Thr Ile Leu Cys
215 220 225 230
Val Lys Gly Lys Leu Thr Lys His Asp Gly Thr Pro Leu Ser Ser Glu
235 240 245
Glu Met Thr Ala Ala Phe Asn Ala Gly Trp Ile Val Ala Asp Asn Asn
250 255 260
Pro Thr Thr Tyr Tyr Pro Val Leu Val Asn Phe Asn Ser Asn Asn Tyr
265 270 275
Thr Tyr Asp Asn Gly Tyr Thr Pro Lys Asn Lys Ile Glu Arg Asn His
280 285 290
Lys Tyr Asp Ile Lys Leu Thr Ile Thr Gly Pro Gly Thr Asn Asn Pro
295 300 305 310
Glu Asn Pro Ile Thr Glu Ser Ala His Leu Asn Val Gln Cys Thr Val
315 320 325
Ala Glu Trp Val Leu Val Gly Gln Asn Ala Thr
330 335
SEQ ID NO: 15
Sequence Length: 348
Sequence Type: Amino acid

Topology: Linear
Molecular Type: Protein
Feature: Amino acid sequence of finbrillin of
Porphyromonas gingivalis OMZ409
Sequence:
Met Val Leu Lys Thr Ser Asn Pro Asn Arg Ala Phe Gly Glu Asp Glu
-10 -5 -1 1 5

Ser Lys Val Ala Lys Leu Thr Val Met Val Tyr Asn Gly Glu Gln Gln
10 15 20
Glu Ala Ile Lys Ser Ala Glu Asn Ala Thr Lys Val Glu Asp Ile Lys
25 30 35
Cys Ser Ala Gly Gln Arg Thr Leu Val Val Met Ala Asn Thr Gly Ala
40 45 50
Met Glu Leu Val Gly Lys Thr Leu Ala Glu Val Lys Ala Leu Thr Thr
55 60 65 70
Glu Leu Thr Ala Glu Asn Gln Glu Ala Thr Gly Leu Ile Met Thr Ala
75 80 85
Glu Pro Val Asp Val Thr Leu Val Ala Gly Asn Asn Tyr Tyr Gly Tyr
90 95 100
Asp Gly Ser Gln Gly Gly Asn Gln Ile Ser Gln Asp Thr Pro Leu Glu
105 110 115
Ile Lys Arg Val His Ala Arg Met Ala Phe Thr Glu Ile Lys Val Gln
120 125 130
Met Ser Pro Ser Tyr Val Asn Lys Tyr Asn Phe Ala Pro Glu Asn Ile
135 140 145 150
Tyr Ala Leu Val Ala Lys Lys Glu Ser Asn Leu Phe Gly Ala Ser Leu
155 160 165
Ala Asn Ser Asp Asp Ala Tyr Leu Thr Gly Ser Leu Thr Asn Phe Asn
170 175 180
Gly Ala Tyr Ser Pro Ala Asn Tyr Thr His Val Asp Trp Leu Gly Arg
185 190 195
Asp Tyr Thr Glu Pro Ser Asn Asn Ala Pro Gln Gly Phe Tyr Val Leu
200 205 210
Glu Ser Thr Tyr Ala Gln Asn Ala Gly Leu Arg Pro Thr Ile Leu Cys
215 220 225 230
Val Lys Gly Lys Leu Thr Lys His Asp Gly Thr Pro Leu Ser Ser Glu
235 240 245
Glu Met Thr Ala Ala Phe Asn Ala Gly Trp Ile Val Ala Asp Asn Asn
250 255 260
Pro Thr Thr Tyr Tyr Pro Val Leu Val Asn Phe Asn Ser Asn Asn Tyr
265 270 275

Thr Tyr Asp Asn Gly Tyr Thr Pro Lys Asn Lys Ile Glu Arg Asn His
    280                 285                 290
Lys Tyr Asp Ile Lys Leu Thr Ile Thr Gly Pro Gly Thr Asn Asn Pro
295                 300                 305                 310
Glu Asn Pro Ile Thr Glu Ser Ala His Leu Asn Val Gln Cys Thr Val
                315                 320                 325
Ala Glu Trp Val Leu Val Gly Gln Asn Ala Thr Trp
            330         -       335
SEQ ID NO: 16
Sequence Length: 350
Sequence Type: Amino acid
Topology: Linear
Molecular Type: Protein
Feature: Amino acid sequence of finbrillin of
Porphyromonas gingivalis ATCC49417
Sequence:
Met Val Leu Lys Thr Ser Asn Pro Asn Arg Ala Phe Gly Asn Ala Gly
-10                 -5                  -1  1               5
Asp Glu Ala Lys Val Ala Lys Leu Thr Val Met Val Tyr Lys Gly Glu
            10                  15                  20
Gln Gln Glu Ala Ile Lys Ser Ala Glu Asn Ala Thr Lys Val Glu Asn
        25                  30                  35
Ile Lys Cys Ser Ala Gly Gln Arg Thr Leu Val Val Met Ala Asn Thr
    40                  45                  50
Gly Gly Met Glu Leu Ala Gly Lys Thr Leu Ala Glu Val Lys Ala Leu
 55                 60                  65                  70
Thr Thr Glu Leu Thr Glu Gly Asn Gln Glu Ala Ala Gly Leu Ile Met
                75                  80                  85
Thr Ala Glu Pro Val Glu Val Thr Leu Val Ala Gly Asn Asn Tyr Tyr
            90                  95                  100
Gly Tyr Asp Gly Ser Gln Gly Gly Asn Gln Ile Ser Gln Asp Thr Pro
        105                 110                 115
Leu Glu Ile Lys Arg Val Arg Ala Arg Ile Ala Phe Thr Lys Ile Glu
    120                 125                 130
Val Thr Met Ser Gln Ser Tyr Ala Asn Lys Tyr Asn Phe Ala Pro Glu
135                 140                 145                 150
Asn Ile Tyr Ala Leu Val Ala Lys Lys Lys Ser Asn Leu Phe Gly Ala
                155                 160                 165
Ser Leu Ala Asn Asn Asp Asp Ala Tyr Leu Thr Gly Ser Leu Thr Thr
            170                 175                 180
Phe Asn Gly Ala Tyr Thr Pro Ala Asn Tyr Thr His Val Asp Trp Leu
        185                 190                 195

Gly Arg Asp Phe Thr Glu Pro Ser Asn Asn Ala Pro Gln Gly Phe Tyr
200 205 210
Val Leu Glu Ser Thr Tyr Ala Gln Asn Ala Gly Leu Arg Pro Thr Ile
215 220 225 230
Leu Cys Ile Lys Gly Lys Leu Thr Lys His Asp Gly Thr Pro Leu Ser
235 240 245
Ser Glu Glu Met Thr Ala Ala Phe Asn Ala Gly Trp Ile Val Ala Asn
250 . 255 260
Asn Asp Pro Thr Thr Tyr Tyr Pro Val Leu Val Asn Phe Glu Ser Asn
265 270 275
Asn Tyr Thr Tyr Thr Gly Glu Ala Val Glu Lys Gly Lys Ile Val Arg
280 285 290
Asn His Lys Phe Asp Ile Asn Leu Thr Ile Thr Gly Pro Gly Thr Asn
295 300 305 310
Asn Pro Glu Asn Pro Ile Thr Glu Ser Ala Asn Leu Asn Val Asn Cys
315 320 325
Val Val Ala Ala Trp Lys Gly Val Val Gln Asn Val Ile Trp
330 335 340
SEQ ID NO: 17
Sequence Length: 353
Sequence Type: Amino acid
Topology: Linear
Molecular Type: Protein
Feature: Amino acid sequence of finbrillin of
Porphyromonas gingivalis 6/26
Sequence:
Met Val Leu Lys Thr Ser Asn Pro Asn Arg Ala Phe Gly Asn Ala Gly
-10 -5 -1 1 5
Asp Glu Ala Lys Val Ala Lys Leu Thr Val Met Val Tyr Lys Gly Glu
10 15 20
Gln Gln Glu Ala Ile Lys Ser Val Glu Asn Ala Ile Lys Val Glu Asn
25 30 35
Ile Lys Cys Gly Ala Gly Gln Arg Thr Leu Val Val Met Ala Asn Thr
40 45 50
Gly Gly Met Glu Leu Ala Gly Lys Thr Leu Ala Glu Val Lys Ala Leu
55 60 65 70
Thr Thr Glu Leu Thr Glu Gly Asn Gln Glu Ala Ala Gly Leu Ile Met
75 80 85
Thr Ala Glu Pro Val Glu Val Thr Leu Val Ala Gly Asn Asn Tyr Tyr
90 95 100
Gly Tyr Asp Gly Ser Gln Gly Gly Asn Gln Ile Ser Gln Gly Thr Pro
105 110 115

Leu Glu Ile Lys Arg Val His Ala Arg Ile Ala Phe Thr Lys Ile Glu
    120                 125                 130
Val Thr Met Ser Gln Ser Tyr Ala Asn Lys Tyr Asn Phe Ala Pro Glu
135                 140                 145                 150
Asn Ile Tyr Ala Leu Val Ala Lys Lys Lys Ser Asn Leu Phe Gly Ala
                155                 160                 165
Ser Leu Ala Asn Ser Asp Asp Ala Tyr Leu Thr Gly Ser Leu Thr Thr
            170                 175                 180
Phe Asn Gly Ala Tyr Ser Pro Ala Asn Tyr Thr His Val Asp Trp Leu
        185                 190                 195
Gly Arg Asp Tyr Thr Glu Ile Gly Ala Ala Thr Val Asn Thr Pro Lys
    200                 205                 210
Gly Phe Tyr Val Leu Glu Ser Thr Tyr Ala Gln Asn Ala Gly Leu Arg
215                 220                 225                 230
Pro Thr Ile Leu Cys Val Lys Gly Lys Leu Thr Lys His Asp Gly Thr
                235                 240                 245
Ala Leu Ser Ser Glu Glu Met Thr Ala Ala Phe Asn Ala Gly Trp Ile
            250                 255                 260
Val Ala Asn Asn Asp Pro Thr Thr Tyr Tyr Pro Val Leu Val Asn Phe
        265                 270                 275
Glu Ser Asn Asn Tyr Thr Tyr Thr Gly Glu Ala Val Glu Lys Gly Lys
    280                 285                 290
Ile Val Arg Asn His Lys Phe Asp Ile Asn Leu Thr Ile Thr Gly Pro
295                 300                 305                 310
Gly Thr Asn Asn Pro Glu Asn Pro Ile Thr Glu Ser Ala Asn Leu Asn
                315                 320                 325
Val Asn Cys Val Val Ala Ala Trp Lys Gly Val Val Gln Asn Val Ile
            330                 335                 340
Trp
SEQ ID NO: 18
Sequence Length: 360
Sequence Type: Amino acid
Topology: Linear
Molecular Type: Protein
Feature: Amino acid sequence of finbrillin of
Porphyromonas gingivalis HG564
Sequence:
Met Glu Thr Asp Ala Thr Val Ser Phe Ile Ile Lys Ser Gly Glu Gly
        -15                 -10                 -5
Arg Ala Val Gly Asp Gly Leu Ala Asp Ala Lys Ile Thr Lys Leu Thr
 -1   1               5                  10                  15

Ala Met Val Tyr Ala Gly Gln Ile Gln Glu Gly Ile Lys Thr Val Glu
                20                  25                  30
Glu Ala Asp Gly Val Leu Lys Val Glu Gly Ile Pro Cys Lys Ser Gly
            35                  40                  45
Ala Asn Arg Val Leu Val Val Val Ala Asn His Asn Tyr Glu Leu Thr
        50                  55                  60
Gly Lys Ser Leu Asn Glu Val Glu Ala Leu Thr Thr Ser Leu Thr Ala
    65                  70                  75
Glu Asn Gln Asn Ala Lys Asn Leu Ile Met Thr Gly Lys Ser Ala Ala
80                  85                  90                  95
Phe Thr Ile Lys Pro Gly Ser Asn His Tyr Gly Tyr Pro Asp Gly Thr
                100                 105                 110
Thr Ser Asp Asn Leu Val Ser Ala Gly Thr Pro Leu Ala Val Thr Arg
            115                 120                 125
Val His Ala Gly Ile Ser Phe Ala Gly Val Glu Val Asn Met Ala Thr
        130                 135                 140
Gln Tyr Gln Asn Tyr Tyr Ser Phe Asn Pro Ala Asp Ala Lys Ile Ala
    145                 150                 155
Ala Leu Val Ala Lys Lys Asp Ser Lys Ile Phe Gly Asn Ser Leu Val
160                 165                 170                 175
Ser Asn Thr Asn Ala Tyr Leu Tyr Gly Val Gln Thr Pro Ala Gly Leu
                180                 185                 190
Tyr Thr Pro Asp Ala Ala Gly Glu Thr Tyr Glu Leu Glu Ala Ser Leu
            195                 200                 205
Asn Thr Asn Tyr Ala Val Gly Ala Gly Phe Tyr Val Leu Glu Ser Lys
        210                 215                 220
Tyr Asp Ala Ser Asn Glu Leu Arg Pro Thr Ile Leu Cys Ile Tyr Gly
    225                 230                 235
Lys Leu Leu Asp Lys Asp Gly Asn Pro Leu Thr Glu Pro Ala Leu Thr
240                 245                 250                 255
Asp Ala Ile Asn Ala Gly Phe Cys Asp Gly Asp Gly Thr Thr Tyr Tyr
                260                 265                 270
Pro Val Leu Val Asn Tyr Asp Gly Asn Gly Tyr Ile Tyr Ser Gly Ala
            275                 280                 285
Ile Thr Gln Gly Gln Asn Lys Ile Val Arg Asn Asn His Tyr Lys Ile
        290                 295                 300
Thr Leu Asn Ile Thr Gly Pro Gly Thr Asn Thr Pro Glu Asn Pro Gln
    305                 310                 315
Pro Val Gln Ala Asn Leu Asn Val Thr Cys Gln Val Thr Pro Trp Val
320                 325                 330                 335
Val Val Asn Gln Ala Ala Thr Trp
                340
SEQ ID NO:  19
Sequence Length:  30

Sequence Type: Nucleic acid
Topology: Linear
Molecular Type: Synthetic DNA
Sequence:
AATTGGATCC GCGCAGCAAG GCCAGCCCGG 30
SEQ ID NO: 20
Sequence Length: 30
Sequence Type: Nucleic acid
Topology: Linear
Molecular Type: Synthetic DNA
Sequence:
AGAGGGATCC GAGCGAACCC CGCTCCCTGT 30
SEQ ID NO: 21
Sequence Length: 23
Sequence Type: Amino acid
Topology: Linear
Molecular Type: Hypothetical
Feature:
X01=Asn or Lys; X02=Ala or Val; X03=Thr or
Ile; X04=Asp or Asn; X05=Ser or Gly
Sequence:
X01 Gly Glu Gln Gln Glu Ala Ile Lys Ser X02 Glu Asn Ala X03 Lys
5 10 15
Val Glu X04 Ile Lys Cys X05
20
SEQ ID NO: 22
Sequence Length: 29
Sequence Type: Amino acid
Topology: Linear
Molecular Type: Hypothetical
Feature:
X01=Asp or Asn; X02=Ser or Gly; X03=Ala, Glu or
Gly; X04=Glu or Lys; X05=Val or Ala
Sequence:
Glu X01 Ile Lys Cys X02 Ala Gly Gln Arg Thr Leu Val Val Met Ala
5 10 15

Asn Thr Gly X03 Met X04 Leu X05 Gly Lys Thr Leu Ala
20 25
SEQ ID NO: 23
Sequence Length: 31
Sequence Type: Amino acid
Topology: Linear
Molecular Type: Hypothetical
Feature:
X01=Val or Ala; X02=Ala or Glu; X03=Glu or Gly
Sequence:
X01 Gly Lys Thr Leu Ala Glu Val Lys Ala Leu Thr Thr Glu Leu Thr
5 10 15
X02 X03 Asn Gln Glu Ala Ala Gly Leu Ile Met Thr Ala Glu Pro
20 25 30
SEQ ID NO: 24
Sequence Length: 13
Sequence Type: Amino acid
Topology: Linear
Molecular Type: Hypothetical
Feature:
X01=Asn or Ser; X02=Thr, Asp or Lys; X03=Ala,
Ser or Asp; X04=Gln or Ala; X05=Asn or Ser
Sequence:
Gln Gly Phe Tyr Val Leu Glu X01 X02 Tyr X03 X04 X05
5 10
SEQ ID NO: 25
Sequence Length: 18
Sequence Type: Amino acid
Topology: Linear
Molecular Type: Hypothetical
Feature:
X01=Gly or Pro; X02=Thr or Lys; X03=Phe or
Tyr; X04=Asn, Glu or Asp; X05=Ser or Gly; X06=
Asn or Gly; X07=Thr or Ile
Sequence:
X01 X02 Thr Tyr Tyr Pro Val Leu Val Asn X03 X04 X05 Asn X06 Tyr
5 10 15
X07 Tyr

SEQ ID NO: 26
Sequence Length: 34
Sequence Type: Amino acid
Topology: Linear
Molecular Type: Hypothetical
Feature:
X01=Ser or Asn; X02=Asn or Gly
Sequence:
Ser Asn Asn Tyr Thr Tyr Asp X01 X02 Tyr Thr Pro Lys Asn Lys Ile
5 10 15
Glu Arg Asn His Lys Tyr Asp Ile Lys Leu Thr Ile Thr Gly Pro Gly
20 25 30
Thr Asn
SEQ ID NO: 27
Sequence Length: 37
Sequence Type: Amino acid
Topology: Linear
Molecular Type: Hypothetical
Feature:
X01=Asn or Thr; X02=Ile or Gln; X03=His or Asn
Sequence:
Ile Thr Gly Pro Gly Thr Asn X01 Pro Glu Asn Pro X02 Thr Glu Ser
5 10 15
Ala X03 Leu Asn Val Gln Cys Thr Val Ala Glu Trp Val Leu Val Gly
20 25 30
Gln Asn Ala Thr Trp
35
SEQ ID NO: 28
Sequence Length: 17
Sequence Type: Amino acid
Topology: Linear
Molecular Type: Hypothetical
Feature:
X01=Thr or Asn; X02=Ser or Thr; X03=Thr or Ala
Sequence:
Thr Gly Ser Leu Thr X01 Phe Asn Gly Ala Tyr X02 Pro Ala Asn Tyr
5 10 15
X03
SEQ ID NO: 29

Sequence Length: 18
Sequence Type: Amino acid
Topology: Linear
Molecular Type: Synthetic Peptide
Sequence:
Glu Leu Thr Ala Glu Asn Gln Glu Ala Ala Gly Leu Ile Met Thr Ala
                5                   10                  15
Glu Pro
SEQ ID NO: 30
Sequence Length: 14
Sequence Type: Amino acid
Topology: Linear
Molecular Type: Synthetic Peptide
Sequence:
Gly Ser Leu Thr Thr Phe Asn Gly Ala Tyr Ser Pro Ala Asn
                5                   10
SEQ ID NO: 31
Sequence Length: 15
Sequence Type: Amino acid
Topology: Linear
Molecular Type: Synthetic Peptide
Sequence:
Thr Tyr Tyr Pro Val Leu Val Asn Phe Asn Ser Asn Asn Tyr Thr
                5                   10                  15
SEQ ID NO: 32
Sequence Length: 17
Sequence Type: Amino acid
Topology: Linear
Molecular Type: Synthetic Peptide
Sequence:
Pro Gly Thr Asn Asn Pro Glu Asn Pro Ile Thr Glu Ser Ala His Leu
                5                   10                  15
Asn

## Claims

1. DNA coding for the fimbrillin protein of *Porphyromonas gingivalis* strains ATCC33277, BH18/10, HW24D-1, OMZ314, OMZ409, ATCC49417, 6/26 or HG564, which is included in the base sequences represented by any of Sequence Nos. 2 through 9.

2. DNA having any base sequence forming a region comprising at least 10 contiguous bases, with at least 50% base sequence homology between at least 2 different base sequences of the base sequences listed as Sequence Nos. 1 through 9.

3. DNA according to claim 2, wherein said homology is at least 70%.

4. DNA according to claim 3, wherein said homology is at least 90%.

5. DNA according to claim 4, wherein the length of the homologous region consisting of said contiguous bases is at least 30 bases.

6. DNA having any base sequence forming a region comprising at least 10 contiguous bases, with less than 50% base sequence homology between at least 2 different base sequences of the base sequences listed as Sequence Nos. 1 through 9.

7. DNA according to claim 6, wherein said homology is 30% or less.

8. A fimbrillin protein of *Porphyromonas gingivalis* having an amino acid sequence represented by any of Sequence Nos. 11 through 18.

9. A peptide having any amino acid sequence forming a region comprising at least 5 contiguous amino acids, with at least 50% amino acid sequence homology between at least 2 different amino acid sequences of the amino acid sequences listed as Sequence Nos. 10 through 18.

10. A peptide according to claim 9, wherein said homology is at least 70%.

11. A peptide according to claim 10, wherein said homology is at least 90%.

12. A peptide having any amino acid sequence forming a region comprising at least 5 continuously linked amino acids, with less than 50% amino acid sequence homology between at least 2 different amino acid sequences of the amino acid sequences listed as Sequence Nos. 10 through 18.

13. A peptide according to claim 12, wherein said homology is 40% or less.

14. An expression vector comprising DNA according to any of claims 1 to 7.

15. A host possessing an expression vector according to claim 14.

16. A peptide consisting of at least 5 contiguous amino acids of any of the amino acid sequences listed as Sequence Nos. 21 through 28.

17. A peptide having any of the amino acid sequences listed as Sequence Nos. 29 through 32.

18. A complex formed by binding a carrier protein with a peptide according to any of claims 9 through 13.

19. A complex formed by binding a carrier protein with a peptide according to claim 16.

20. A composite antigen formed by binding a carrier protein with a peptide according to claim 17.

21. An antibody against a peptide or protein according to any of claims 8 to 13 or 16 to 19.

22. An agent for the prevention or improvement of periodontal diseases which contains an antibody according to claim 21.
